# EUROPEAN PATENT APPLICATION

(11) **EP 4 721 780 A1**
(43) Date of publication of application: **08.04.2026**
(21) Application number: 24203834.7
(22) Date of filing: 01.10.2024
(51) Int. Cl.: A61M 1/32, A61M 1/36

(54) **BLOOD FOAM MANAGEMENT APPARATUS, SYSTEM AND METHOD**

(71) Applicant: Sangair AB, 171 65 Solna (SE)
(72) Inventor: SJÖHOLM, Johan, 224 78 Lund (SE); RUNDGREN, Henrik, 171 65 Solna (SE)
(74) Representative: KIPA AB

(57) **Abstract**

A foam management system (1000), apparatus (300), and method are disclosed for a foamed blood product , the apparatus comprising an antifoaming device (400) for receiving a pressurized foam (60) from a pressurized foamed blood product, said antifoaming device allowing a gas (68) to be separated from said foam (60), wherein said antifoaming device converts said pressurized foam (60) into said blood product, allowing the blood product to be recovered from said foam (60), substantially without the foam.

## Description

### TECHNICAL FIELD

The present disclosure relates to a foam management system, apparatus and method for a blood product, whereby foam is converted back to the blood product, preventing loss of blood volume due to removal of foam. More particularly, the present disclosure relates to a foam management system, apparatus and method for ozonated blood that provides for efficient return of ozonated blood to the body, requiring minimal blood volume to be disposed due to foaming.

### BACKGROUND

It is known that ozone is a powerful oxidizing agent that can be used to inactivate microbial agents in blood, such as viruses and bacteria, that for example can be responsible for sepsis. Thus, ozonating blood provides an effective strategy for treatment of sepsis, where the patient is unable to receive conventional treatment, such as allergy to antibiotics such as penicillin or where it is more effective to treat directly through ozonation. Ozonation provides a treatment for sepsis that is different from conventional approaches to using antibiotics or antivirals, thus providing an alternative treatment for severe cases or cases resistant to conventional treatment.

The problem with conventional approaches to treating blood with ozone is that during ozonation of blood, there is resultant foaming of blood. The foamed blood cannot be returned to the body and is discarded. The remaining ozonated blood is returned to the body. Thus, ozonation of blood to treat sepsis creates foam that cannot be returned to the body and must be discarded. This can result in around 4dl of foam and this is equivalent to 0.4L of blood, which is around 10% or more of the total blood volume of a patient that must be removed, in a patient that is already ill with sepsis. Thus, previous disclosures in the art have a disadvantage in that they are unable to return the volume of blood associated with foam back to the patient. This creates stress on the patient's body if you remove high amounts of blood from the patient, which is inherent when you remove foam. Additionally, the patient may require additional blood transfusions, which may place an economic burden on the patient and/or the healthcare system.

WO2008066470A1 teaches an apparatus and method for ozone treatment of liquid foodstuff. The disclosure relates to a method for inhibiting bacterial growth in liquid media by means of ozone containing gas flow, whereby a liquid medium at ambient temperature is passed by a finely divided gas stream containing ozone, the liquid medium is passed to a dwell time space while being mixed to provide complete mixing between liquid and ozone, whereupon the liquid medium is degassed to eliminate excess of ozone dissolved therein. However, the disclosure does not teach an effective way to treat blood through ozonation.

EP3193948A4 discloses an apparatus and method for contacting blood with ozone. The apparatus, system, and method describe contacting blood with ozone to kill microorganisms in the blood. The method involves injecting microbubbles of ozone containing gas into a flow of blood, preferably at a temperature of less than 12°C. The apparatus includes a blood flow conduit including a blood ozone contacting portion including a porous ozone injector. The apparatus provides an ozonation chamber that requires flow of blood vertically upwards through the ozonation chamber, providing a desired effect of reduced foaming. However, even though the inventors have developed an apparatus that reduces foaming, the foamed blood that is inherently formed through the ozonation process in the ozonation chamber is directed to a collection bag that is then discarded. This results in reduced total volume of blood in the patient that is already under treatment for a serious illness. This can add to weakness and lead to a more prolonged recovery time. Thus, the inventors of this disclosure do not recognize the need for defoaming foamed blood or the advantages thereof.

US7131966B1 to Tamari, discloses an apparatus to trap and remove air bubbles in a venous side of cardiopulmonary bypass circuit, where they are using a suction means to remove air from the blood chamber. The invention is a reservoir that incorporates automated means to remove air bubbles from the venous line before blood enters the arterial blood pump. In another form, the reservoir includes means that handles foam prior to the blood entering the blood pump. Tamari, also discloses that blood loss in liquid form is prevented from exiting outlet of tube, blood in the form of foam may still do so. Tamari discloses that to limit blood foam from exiting the top of tube, a defoamer means which is a defoamer sponge preferably incorporating anti-foam A, is used to collapse the blood foam into liquid that then flows back down in blood chamber. In the disclosure of Tamari the defoamer at the top of tube provides the desirable defoaming action while limiting contact between the defoamer and the blood. However, the invention as disclosed relies on suction to remove air from the blood chamber. More specifically, the disclosure provides a Purge tube to allow gas to be purged and an air-venting tube, where the same suction is applied to both.

Therefore, the disclosure of Tamari provides an active means to remove air from the blood chamber and teaches defoaming using a sponge, thus using a physical means by physically creating a barrier that collapses foam into liquid. Additionally, using an active suction to remove air bubbles can result in further complications such as blood loss from foam being suctioned, as disclosed in Tamari.. This requires further intervention, such as a sponge to physically collapse the foam and using antifoam agents, that can embolize into blood, as disclosed by Tamari. Additionally, the system of Tamari does not involve microbubbles that are injected into the blood flow and there is disclosure in Tamri for removing foam that formed from dissolved microbubbles. Thus, Tamari does not recognize the need for removing foam formed from dissolved microbubbles or a simpler and/or passive means to separate such a foam from blood.

Therefore, there is a need for a system that minimizes and/or reduces the amount of blood lost due to foaming, from a medical procedure. As such there is a need to provide a system that manages the foam generated as a result of the treatment, for example using a passive means, in order to remove gas from foam to convert it into blood, that does not require the complicated apparatus disclosed by the one or more prior art, as noted above. Additionally, there is a need for a system that provides a means to separate foam from the blood and convert the foam into blood, where the foam comprises microbubbles that are dissolved substantially in blood.

Therefore, in one such example, there is a need for a new system that efficiently ozonates blood for deactivating microbes for treatment of infection such as sepsis while also minimizing and reducing the amount of blood lost due to foaming, for example, from the ozonation procedure. Thus, a system is required that is relatively simple to implement that is targeted towards not just reducing foaming but rather eliminating the foam from the ozonated blood, that will allow for maximal return of ozonated blood volume back to the patient after ozonation where the foam is formed from microbubbles dissolved in blood and/or where the system uses a passive means to remove gas from foam to convert it into blood. Hence, an improved system for is needed for ozonating blood to treat for microbes to treat sepsis and to remove the foam, thereby defoaming the ozonated blood so it can be returned to the patient, thereby maximizing the return of blood volume back to the body. Such a device or system would improve efficiency of ozonation and returning ozonated blood to the body, hence improving procedural efficiency and efficacy. There will be a greater volume of ozonated blood, that is returned to the patient, maximizing deactivation of microbes such as bacteria and viruses among others, and increasing efficacy of sepsis treatment.

### SUMMARY

It is an object of the present disclosure to provide a system for foam management and a method for ozonating blood, to return a maximum volume of ozonated blood to the patient with minimal loss of blood volume, in order to effectively deactivate microbes in the blood, such as bacteria or viruses.

Accordingly, embodiments of the present disclosure preferably seek to mitigate, alleviate or eliminate one or more deficiencies, disadvantages or issues in the art, such as the above-identified problems with foaming of ozonated blood, singly or in any combination by providing a device/foam management apparatus/system, and a method, that defoam the ozonated blood and then treat the foam to allow the secondary ozonated blood and gas to separate, allowing secondary ozonated blood to be returned to the body, according to the appended patent claims.

Thus, inventors of the present disclosure have developed a system that allows foam management during a medical procedure such as ozonation of blood. In some such examples, the ozonation of blood is performed under pressure where the blood is pressurized and/or the ozone is injected into the blood under pressure, where the foam comprises microbubbles that are dissolved in blood. In some embodiments of the present disclosure, the system provides for a passive means to remove the trapped microbubbles in the foam. Thus, the system may provide increased efficacy of treatment of microbes in the blood, such as bacteria, viruses and others, that relies on managing foam by (i) defoaming the pressurized foamed ozonated blood and (ii) and antifoaming the pressurized foam to convert is back to ozonated blood, rather than removing the foam, which allows a greater volume of ozonated blood to be returned to the body. Additionally, by treating the foamed blood in order to defoam it, it allows secondary ozonated blood to be returned to the body, as the foamed blood contains a higher volume of ozone. The system allows for passively converting the foam that comprises microbubbles of dissolved ozone, back into blood, utilizing the pressures already present in the system. This can increase treatment efficacy and reduce procedural complexity by allowing one or more of several advantages: by allowing the treatment duration to be shortened, and/or improved recovery time, and/or more effective ozonation of blood, and/or increased effectiveness of deactivation of microbes in the blood, leading to improved outcomes for patients.

According to aspects of the disclosure a method and a foam management apparatus and/or system are disclosed comprising: antifoaming device for receiving a pressurized foam from a pressurized foamed blood product, said antifoaming device allowing a gas to be separated from said foam, wherein said antifoaming device converts said pressurized foam into said blood product, allowing the blood product to be recovered from said foam in the system, substantially without the foam. In one such example, the present method, apparatus and/or system allow the blood product to be recovered passively from said foam utilizing the pressures in the system, wherein the blood product is recovered substantially without the foam and/or wherein the foam comprises dissolved microbubbles of gas. In some such examples, the gas comprises ozone. In other examples the gas may comprise oxygen.

According to a first aspect of the present disclosure the method and the foam management apparatus and/or system further comprises: a defoaming device for receiving a pressurized foamed blood product, for separating a pressurized foam therefrom for processing, to enable return of the blood product to a person, substantially without the foam. In one such example, the present method, apparatus and/or system allow the pressurized foam to be separated from the blood product passively utilizing the pressures in the system, wherein the blood product is recovered substantially without the foam, and/or wherein the foam comprises dissolved microbubbles of gas. In some such examples, the gas comprises ozone. In other examples the gas may comprise oxygen.

According to additional aspects of the disclosure, a method, a foam management apparatus and system, are disclosed for ozonating blood and returning a maximum volume of ozonated blood to the patient, ozonated blood is returned to the patient and the resulting foam from ozonation is not discarded but rather processed to allow secondary ozonated blood and gas to separate, allowing the secondary ozonated blood from the foam to also be returned to the patient.

According to another aspect of the present disclosure, a foam management apparatus is disclosed for managing the foam in foamed ozonated blood, the foam management apparatus comprising:
(a) a defoaming device for receiving the pressurized foamed ozonated blood for separating a foam and/or pressurized foam, therefrom for processing, to enable return of a primary ozonated blood from said foamed ozonated blood back to the body, said foam being pressurized. In some instances, this can be viewed as a first pass of the procedure where ozonated blood (primary ozonated blood) is returned to the patient.
(b) An antifoaming device whereby said antifoaming device allows processing of said pressurized foam to retrieve remaining secondary ozonated blood therefrom for return to the body, whereby said pressurized foam is converted into the secondary ozonated blood.

The advantage of such a foam management apparatus is that it allows defoaming of pressurized foamed ozonated blood by the defoaming device by separating the pressurized foam from the ozonated blood, allowing ozonated blood to be returned to the body without the foam. Furthermore, the apparatus allows the antifoaming device to process the foam to further convert the pressurized foam into more and/or highly ozonized blood, that can also be returned back to the body. In some instances, this can be considered a second pass of the procedure where the removed pressurized foam is processed, so it can be returned to the patient as ozonated blood (the secondary ozonated blood). Thus, the foam management apparatus enables substantially all of the blood that has been removed from the body to be ozonated to be returned back to the body in the form of ozonized blood. As such the foam management apparatus eliminates the need for discarding foam, by which blood can also be removed that is contained in the foam. Thus, the blood volume of the patient is maintained, and the patient substantially does not experience loss of blood volume due to the procedure. This increases procedural effectiveness and efficacy in eliminating pathogens from the blood while substantially maintaining blood volume. Additionally, the foam which would previously in the prior art have been discarded is now processed to convert it to more ozonized blood which has more ozone content than ozonated blood which was previously removed when defoaming. This more ozonized blood further increases procedural effectiveness and efficacy at removing bacteria or viruses or the like, from the blood. Additionally, now more ozonated blood can be returned to the patient than in conventional procedures where pressurized foam is converted passively into blood product comprising ozonated blood, by utilizing the pressures that are already in the system. Thus, the procedural efficiency is enhanced for treating conditions such as sepsis. In some such examples, the pressurized foam comprises microbubbles of dissolved ozone and/or wherein said microbubbles are dissolved substantially in said blood.

As a feature of this aspect, more specifically, as it relates to the antifoaming device of the foam management system, it is designed for receiving the pressurized foam from the defoaming device, the antifoaming device comprising an egress to allow a gas to be separated from said foam, allowing said foam to be converted into more ozonated blood. Since the foam is pressurized as the ozonation of the blood from the patient's is done under pressure, the pressurized foam enters the bag under pressure, where there is an outlet or egress for vapor or gas to leave, this allows the gas in the foam to be separated from the liquid or blood in the foam. This enables effective separation of blood and allows the foam to be converted into ozonized blood which in some instances is referred to as secondary ozonated blood. Since, foam is pressurized and contains microbubbles of dissolved ozone, it means that the blood that is recovered from the foam is not only ozonized but rather more ozonized, which increases procedural efficacy as it allows more ozonized blood (i.e. the secondary ozonated blood it has greater amount of ozonation than previously removed from the defoaming device which is the primary ozonated blood) to be returned to the patient, making it more effective in destroying viruses and/or or other pathogens, and additionally the pressurized foam can be separated passively from the blood using the pressure it is under. Thus, in some instances it can reduce procedural time for treatment of sepsis and reduce recovery time for the patient. In addition, as foam is no longer discarded and ozonated blood is additionally recovered through antifoaming from the foam that in some examples, comprises microbubbles of dissolved ozone, it allows substantially more ozonated blood to be returned to the patient than through conventional procedures. In some such examples, the microbubbles of ozone dissolved in the blood, may be about 5 microns in size. In such examples the microbubbles of ozone form the foamed ozonated blood, and thus form the foam that is recovered from said foamed ozonated blood. In some such examples, the microbubbles of ozone gas are entrained in the blood or blood flow and have a size and/or diameter of less than about 5µm. In some example the microbubbles are a size of about 2 µm. In some such examples, the size of the microbubbles is between about 2 µm to about 5 µm.

According to a second aspect of the present disclosure, a system is disclosed for ozonating blood in a patient, the system comprising: one more pumps for pressurizing the blood leaving the patient and one or more injectors, for injecting pressurized ozone into the pressurized blood from the one or more pumps. The one or more pumps and/or the one or more injectors resulting in pressurized foamed ozonated blood. The system further comprises a foam management apparatus comprising a defoaming device for receiving the pressurized foamed ozonated blood for separating a foam therefrom, and returning primary ozonated blood to the body, and additionally allowing for processing of said foam by an antifoaming device, to enable return of ozonated blood from said foamed ozonated blood to the body, said foam being pressurized, allowing defoaming of the foamed ozonated blood, as it can travel substantially vertically against gravity from the defoaming device into the antifoaming device, under pressurization. In some such examples, the foamed ozonated blood is under a combined pressure from the one or more pumps and or one or more injectors for injecting microbubbles of ozone containing gas, where the combined pressure is about 1.5 bars and/or less than about 1.5 bars.

Additionally, the foam management apparatus allows processing of said pressurized foam to retrieve (remaining) secondary ozonated blood therefrom for return to the body, thereby antifoaming the foam, whereby said pressurized foam is converted into the secondary ozonated blood. This allows return of return of maximal amount of blood volume to the patient. Thus, the patient substantially does not result in blood volume loss due the procedure itself as the secondary ozonated blood that forms a part of the foam, is also returned to the body along with primary ozonated blood that it initially separated from the foam in the defoaming device. This improves ozonation of the blood and the effectiveness of the procedure as a result in terms of destroying or killing pathogens in the blood such as bacteria, viruses and others. This can thus allow more ozonated blood to be returned to the patient than conventional procedures and can also improve recovery time and reduce patient weakness during treatment for sepsis.

As a feature of this aspect, the system further comprises: an antifoaming device for receiving said pressurized foam, said antifoaming device comprising an egress to allow a gas to be separated from said foam, allowing said foam to be converted into more ozonated blood. Again, the advantage is that the foam that would otherwise have been thrown is reused. After foam is separated from the ozonated blood (primary ozonated blood) in the defoaming device, it is then received by the antifoaming device which allows separation of gas and liquid, i.e. blood, allowing the foam to be converted into ozonated blood that is secondary ozonated as a result of being a part of the foam. In some such examples, the antifoaming device functions passively to separate the pressurized foam into its constituents, the gas and the secondary ozonated blood, under the effect of pressure in the system or in other words as a result of the pressure of the blood in the system, where the blood has microbubbles of ozone dissolved therein. In some such examples, said microbubbles are dissolved substantially in said blood and/or blood product. In additional examples, said microbubbles are dissolved substantially completely in said blood and/or blood product.

This secondary ozonated blood can be then returned to the body, reducing any loss in blood volume as a result of the procedure and increasing procedural efficacy as the blood recovered from within the foam is secondary ozonated and thus even more effective at destroying and/or deactivating pathogens. Thus, substantially more ozonated blood is returned to the patient than in conventional procedures. Thus, making this procedure more effective at treating sepsis.

As another feature of this aspect, the system further comprises one or more processing units to operate said one or more of: the one or more injectors, the one or more pumps, the defoaming device, the antifoaming device and sensors. The use of processing units to operate the pumps, defoaming device and antifoaming device may help regulate the procedure, make it more repeatable and enhance predictability and efficiency of the procedure. It would also allow the operator greater control and ease of use and may make the procedure more comfortable for the patient. In some such embodiments, the one or more processors may allow for automation/automated control, of the system so that the pumps, the defoaming device and antifoaming device operate automatically under directions fed into the processor, allowing for a more efficient delivery of ozone, regulation of the defoaming device as well as the antifoaming device, thus offering automated controls for the system. This may make the procedure faster, and hence easier for the patient to experience or endure and additionally be improve procedural efficiency and efficacy and may aid in a faster recovery from the condition being treated, such as sepsis. Additionally, an advantage follows that this feature of the broad aspect allows more ozonated blood to be returned to the patient than conventional procedures.

As another feature of this broad aspect, the one or more injectors, comprise at least four injectors. As a further example of this, the one or more injectors for injecting pressurized ozone into the blood are operating at pressure of 1 bar. The injectors inject microbubbles of ozone containing gas into a flow of blood, such that they are substantially completely dissolved in the blood. The advantage of injecting ozone under pressure, in combination with additional pressurization from one or more pumps such as a peristaltic pump, allows the blood to be ozonated under pressure, which allows the blood to enter the defoaming device under pressure and thus, counters the effects of gravity and allows a system where flow is self-directed both into a defoaming chamber or chamber of the defoaming device to separate foam from the ozonated blood, which allows ozonated blood to be returned to the body. In some such examples, the combined pressure exerted by the one or more pumps and/or the one or more injectors is equal to and/or less than about 1.5 bars. Furthermore, because the separated foam is also pressurized as a result of the initial ozonation under pressure and the pressurized blood, the foam is also under self-directed flow into the antifoaming device against gravity. In other words, the foam moves passively into the antifoaming device, under the action of pressure in the system from the pressurized blood. This can allow the foam to enter substantially vertically against gravity into the antifoaming device. The pressure ultimately also helps with egress of air or gas from the foam in the antifoaming device, which allows the foam to be converted into ozonated blood, but because of the nature of the foam being more ozonated than primary ozonated blood recovered from the defoaming device, the blood recovered from the foam (secondary ozonated blood) is also more ozonated than the primary ozonated blood. Hence pumping ozone under pressure into the blood offers several advantages, by allowing the flow of foamed and defoamed ozonated blood to be directed into and out of the defoaming device as well as allowing flow of the foam into the antifoaming device and return of secondary ozonated blood therefrom. Thus, in some examples, pressurization of ozone allows for sustaining a self-directed closed system. In some such examples, this closed system can be monitored using the one of more processing units. This also provides all of the advantages outline hereinabove. In one example, this allows more ozonated blood with a greater amount of ozone in the blood, to be returned to the patient than in conventional procedures.

In one broad aspect, a blood management system is provided, said defoaming device further comprises a sensor for checking a level of the ozonated blood in a defoaming chamber or chamber of said defoaming device, wherein if there is mixing of ozonated blood and foam and/or the level of ozonated blood is low, enabling mixing of foam and ozonated blood, a pressure of the foamed ozonated blood can be increased by increasing the pressure of in the one or more pumps and/or the injectors, and thereby increase the level of ozonated blood in the chamber and thereby reduce and/or eliminate mixing of foam and blood.

As still another feature of this broad aspect, the foam management system comprises the sensor in said defoaming device for checking the level of blood in the chamber, wherein the level of ozonated blood in the defoaming chamber or chamber, is an indication for the pressure the blood is under due the one or more pumps and/or ozone injectors. If there is mixing observed between the ozonated blood and foam, then the pressure can be raised in the one or more pumps and/or injectors for injecting ozone into the blood. The sensors permit the regulation of the defoaming device, in that if it is operating at lower than ideal pressure, whereby the foam and ozonated blood are mixing, the level of the ozonated blood in the chamber can indicate that the pressure is lower than ideal.

Thus, in response, the pressure at the one or more pumps and/or injectors can be increased, which will raise the level of ozonated blood in the chamber of the defoaming device and prevent mixing of ozonated blood with the foam. The pressure at which the pump and or the injectors operate creates a myriad of small or tiny bubbles of ozone containing gas in the blood and/or blood flow, or microbubbles which can be less than about 5 microns in size. In some examples, the microbubbles are at a size of about 2 microns. In some embodiments of the present disclosure, by changing the pressure in the blood, the size of the microbubbles can be changed. In some examples of the present disclosure, the one or more injectors inject microbubbles of ozone containing gas into a flow of blood, where the system has both cooling and heat exchanges, and the microbubbles are injected into blood that is at a temperature that is less than body temperature and above the freezing point, wherein the ozonated blood that is returned to the patient and/or one or more blood bags after heat exchange, where it is warmed to a temperature of about body temperature.

This allows for procedural efficacy as ozonated blood and foam can be separated more efficiently for return allowing ozonated blood to be returned to the patient. This may help reduce procedural time and may improve recovery. As an example of this feature, the one or more processing units of the system are configured to operate the sensor for checking the level of blood and thus the pressure in the defoaming chamber or chamber of the defoaming device or evaporator, and thus may allow for a more streamlined assessment of blood levels and thus potential mixing of foam and ozonated blood, allowing the pressure to be increased to mitigate this.

In some such examples, the processor may allow automation/automated control, of this part of the system and may also include other parts of the system, which may ease of burden for the operator as blood levels in the chamber may be monitored automatically by one or more processor that are in communication with both the sensor and the pumps. In an additional broad aspect of the present disclosure, the system comprises any one of the broad aspects and features of the foam management apparatus disclosed herein above.

According to one aspect of the disclosure, a method is provided for ozonating blood using a foam management apparatus comprising a defoaming device, the method comprising the steps of: ozonating blood exiting the patient by injecting ozone into a bloodstream of the patient under pressurization; receiving foamed ozonated blood into the defoaming device, defoaming the foamed ozonated blood in the defoaming device, for example in a chamber or defoaming chamber of the defoaming device, into primary ozonated blood and pressurized foam, processing said pressurized foam by antifoaming it to convert it to secondary ozonated blood for return to the body, and returning primary ozonated blood and secondary ozonated blood back to the body, substantially without the foam. The method provides the same advantages discussed herein above, for broad aspects and features of the foam management apparatus and system for ozonating blood. Some of the main advantages of the method are that it allows utilization of substantially all of the blood that is received from the body. Thus, it allows substantially all of the blood that is received from the body to be returned to the body as ozonated blood. The blood received from the patient that is then ozonated and forms into foamed ozonated blood. Advantageously, the method of the present disclosure prevents the foam from this foamed ozonated blood from being discarded, so that it is separated into ozonated blood (primary ozonated blood) that is returned to the body, and foam that is separated therefrom in the defoaming chamber of the defoaming device under pressurization. Advantageously, this foam can be processed further by antifoaming it (for example by using an antifoaming device) to convert it into ozonated blood, but because of the nature of the foam itself because it has a high content of ozone than primary ozonated blood, the ozonated blood extracted from the foam (secondary ozonated blood) is also more ozonated than primary ozonated blood. Thus, it provides the same advantages discussed herein above, that it helps increase procedural efficacy and efficiency and allows more ozonated blood to be returned to the patient than conventional procedures. In some such examples, the foam is pressurized foam containing microbubbles or ozone containing gas and the antifoaming device separates the gas trapped in the microbubbles, from the blood thereby converting said foam into ozonated blood. In some examples, the antifoaming device separates the gas trapped in the microbubbles, from the blood passively, under the influence of the pressure in the system from the pressurized blood.

According to a feature of this broad aspect, the method of managing foam further comprises the steps of receiving pressurized foam from the defoaming device into an antifoaming device; permitting gas egress from the antifoaming device to allow the secondary ozonated blood and gas to separate, wherein the secondary ozonated blood remains in the antifoaming device; and returning the secondary ozonated blood back to the patient, substantially without the foam. Thus, embodiments of the present disclosure allow for utilization of foam, which would otherwise have been discarded conventionally. Thus, they offer an advantage where the foam instead of being discarded can now be processed in the antifoaming device to remove gas therefrom from the egress, resulting in ozonated blood to be recovered from the foam. Since the foam is more ozonated (i.e. it contains more ozone than the ozonated blood [primary ozonated blood] retrieved from the defoaming device), the blood recovered from the foam is also more ozonated. Thus, advantage of the present method is in allowing substantially all of the blood removed from the body to be returned to the body in ozonated form, which includes more ozonated blood recovered from the foam.

As a feature of this broad aspect, the foam management method further comprises the step of maintaining a pressure of said pressurized foamed ozonated blood 52 by injecting ozone under pressurization using one or more injectors and/or pressurizing the blood using one or more pumps at a combined pressure of at least about 1.5 bar, at a first step 2000. The one or more injectors inject microbubbles of ozone containing gas into a flow of blood. In some such examples, said microbubbles are dissolved substantially in said foamed ozonated blood 52.

As a feature of this method, the method of managing foam further comprises the steps of: sensing a level of ozonated blood in the defoaming device and/or the defoaming chamber or chamber of the defoaming device, and determining if there is mixing of ozonated blood and foam at that level; and if it is determined that there is mixing of foam and/or ozonated blood at that level, and/or if it is determined that the level of ozonated blood in the defoaming device is too low enabling mixing, then pumping foamed ozonated blood into the defoaming device where the foamed ozonated blood is at a greater pressure than before. Thus, pressure is increased in the one or more pumps that pressurize blood leaving the patient and/or in the one or more injectors that inject ozone into the blood from the pump. This provides the advantage of allowing for adjustment and/or optimization of defoaming in the defoaming chamber of the defoaming device, so that the foam and the ozonated blood are substantially adequately separated. This means if the chamber is functioning less optimally than desired, i.e. permitted mixing of foam and ozonated blood, more pressurized foamed ozonated blood can be entered into the chamber to increase procedural efficiency of the treatment, by increasing the pressure of the one or more pumps and/or injectors. This may reduce procedural time for the patient and may increase ease of use for the operator.

As an additional feature of this method, the method of managing foam further comprises ozonating blood received from the patient under pressurization, as discussed previously herein above. The advantage of this is that the pressurization of ozone in the blood received from the patient, enables the process of ozonisation to be completed more smoothly, as the pressurized ozonated blood that contains foam is directed to the chamber or in other words the collection chamber which is in the defoaming device. Here the foam is separated from the ozonated blood (primary ozonated blood) which is also returned to the body with the help of the pressurization. Because the foam, which is as discussed, also under pressure, is guided to the antifoaming device where the pressure additionally aids in separation of gas from the blood, thereby converting the foam into ozonated blood. Also, since the foam has a higher content of ozone, the ozonated blood received from it, also has higher ozone content. Thus, advantages of the present disclosure are that pressurization of ozone facilitates separation of foam from ozonated blood (defoaming) and additionally facilitates processing of foam to remove gas therefrom to recover secondary ozonated blood from the foam (antifoaming). In some such examples, the disclosed embodiments of the ozone injectors inject ozone into the blood under a pressurization of at least one bar. This also provides the same advantages as discussed herein above.

In another broad aspect of the present disclosure, a method is disclosed for ozonating blood, the method using the foam management apparatus and/ or system as described herein, for defoaming the foamed ozonated blood, wherein the ozonated blood and secondary ozonated blood are returned back to the patient substantially without the foam. The advantages are as discussed previously herein above.

In additional broad aspects of the present disclosure, software is disclosed for implementing a foam management system for ozonating blood, that includes code segments for performing the method as disclosed herein above. In additional broad aspects, a computer readable medium is disclosed that comprises having stored thereon the software as disclosed, for processing by one or more processing units. The advantages of such a system would allow ease of use for the user and allow automated control/automation of one or more parts of the system, such as the pumps, the defoaming device, the antifoaming device and the sensor of the defoaming device. This would allow the procedure to be more automated and may provide additional safety and predictability of the procedure for the patient. It may also increase procedural efficiency and reduce both the procedural time and may help reduce the recovery time for the patient.

In some embodiments of the present disclosure, the foam as described herein comprises a mass of small bubbles in liquid, formed on the surface. It is a frothy mass of fine bubbles. In accordance with embodiments of the present disclosure gas is dispersed in a non-gaseous material, for example a liquid such as blood. Foams are two-phase material systems where a gas is disbursed in a second, non-gaseous material, specifically, in which gas cells are enclosed by a distinct liquid or solid material. The foam may contain more or less liquid, according to circumstances, although in the case of gas-liquid foams, in some examples the gas may occupy most of the volume.

It should be emphasized that the term "comprises/comprising" when used in this specification is taken to specify the presence of stated features, integers, steps or components but does not preclude the presence or addition of one or more other features, integers, steps, components or groups thereof.

Further features of, and advantages with, the present disclosure will become apparent when studying the appended claims and the following description. The skilled person realizes that different features of the present disclosure may be combined to create embodiments other than those described in the following, without departing from the scope of the present disclosure.

### BRIEF DESCRIPTION OF THE DRAWINGS

The above, as well as additional objects, features and advantages of the present disclosure, will be better understood through the following illustrative and non-limiting detailed description of exemplary embodiments of the present disclosure, wherein:
Fig. 1 is a side view of a foam management apparatus and system for ozonating blood, illustrating ozone being injected into the blood under pressure, in accordance with embodiments of the present disclosure;
Fig. 2 a side view of a foam management apparatus and system for ozonating blood, wherein the foamed ozonated blood is received within the defoaming device, for removal of foam therefrom, in accordance with an embodiment of the present disclosure;
Fig. 3 is a side view of a foam management apparatus and system for ozonating blood, wherein the foamed ozonated blood is received within the defoaming device, for removal of foam therefrom, in accordance with an embodiment of the present disclosure;
Fig. 4 is a side view of a foam management apparatus and system for ozonating blood, wherein the foam and the ozonated blood are separated, the ozonated blood exiting the chamber for return to the body and the foam exiting the defoaming device for further processing, in accordance with an embodiment of the present disclosure;
Fig. 5A is side view of a foam management apparatus and system for ozonating blood, wherein the foam is received in the antifoaming device for removal of gas therefrom, allowing the more ozonated blood to be retrieved from said foam to be returned to the body, in accordance with an embodiment of the present disclosure;
Fig. 5B is side view of a foam management apparatus and system for ozonating blood, wherein the foam is received in the antifoaming device for removal of gas therefrom, allowing the more ozonated blood to be retrieved from said foam to be returned to the body, in accordance with an embodiment of the present disclosure;
Fig. 6A, 6B and 6C, show an alternative embodiment of a method of foam management, where ozonated blood is delivered to a patient via a blood bag, in accordance with an embodiment of the present disclosure;
Fig. 6D, is an illustration of a flow chart showing a method for foam management of a blood product using an antifoaming device, in accordance with an embodiment of the present disclosure;
Fig. 7A is an illustration of a flow chart showing a method for managing foam, whereby ozonated blood and secondary ozonated blood are both returned to the patient, substantially without the foam, in accordance with an embodiment of the present disclosure;
Fig. 7B is an illustration of a flow chart showing a method for managing foam, wherein secondary ozonated blood from the foam, is returned to the patient substantially without the foam, in accordance with various embodiments of the present disclosure;
Fig. 7C is an illustration of a flow chart showing a method for managing foam, wherein the method comprises sensing a level of ozonated blood in the defoaming device and proceeding accordingly, in accordance with various embodiments of the present disclosure; and
Fig. 7D is an illustration of a flow chart showing a method for managing foam, wherein the method further comprises ozonating blood by injecting ozone under pressurization of at least one bar, in accordance with various embodiments of the present disclosure.

### DETAILED DESCRIPTION OF EXAMPLE EMBODIMENTS OF THE DISCLOSURE

The following description focuses on an embodiment of the present disclosure applicable to a systems and foam management apparatuses for determining ozonating blood and in particular to systems and foam management apparatuses for ozonating blood where substantially the full volume of ozonated blood is returned to the patient, substantially without the foam. However, it will be appreciated that the disclosure is not limited to this application but may be applied to many other applications including for example, allowing line operators to provide a dynamic line rating instead of a static line rating, allowing better use of the line capacity.

While specific embodiments of the disclosure now will be described with reference to the accompanying drawings, the disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements. Like references, characters refer to like elements throughout the description.

### Foam management apparatus

Foam management apparatus as disclosed and described herein, in accordance with some embodiments of the present disclosure, with reference to drawings enclosed herewith:

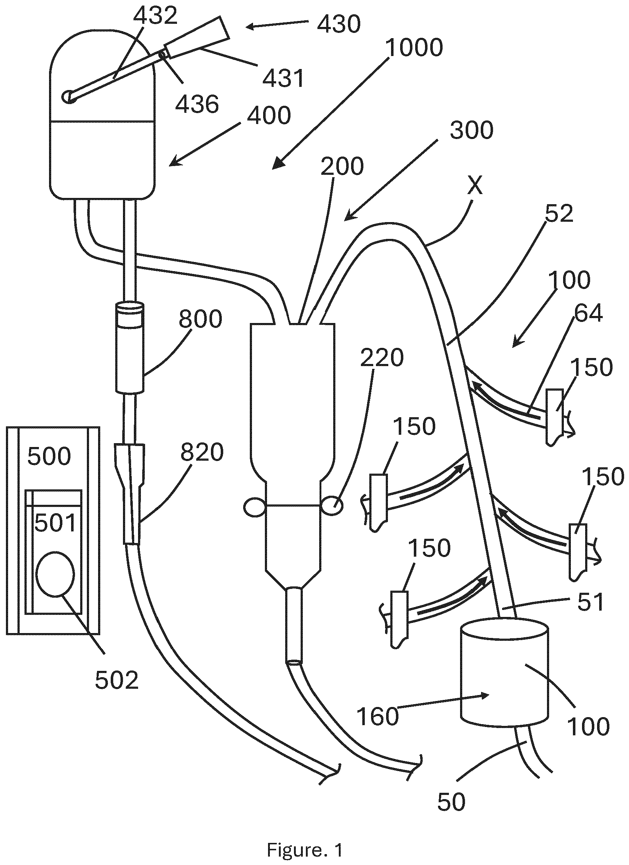

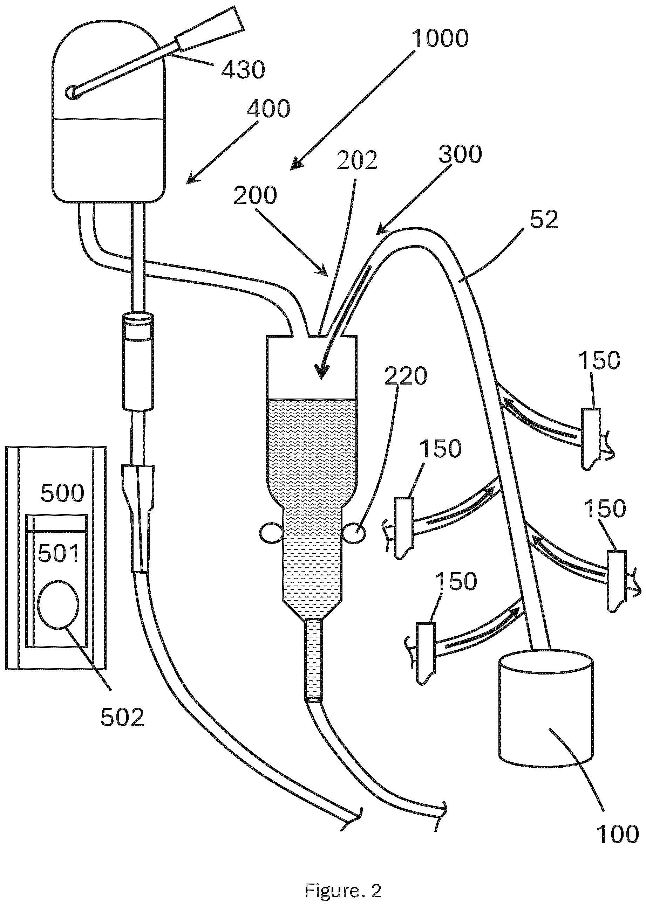

With reference to FIG. 1, in accordance with some embodiments of the present disclosure, an foam management apparatus 300 is shown for defoaming ozonated blood. The foam management apparatus 300 receives blood 50 from the patient. In some such embodiments, the blood exiting the patient is ozonated and enters the defoaming device 200 as pressurized foamed ozonated blood 52 as shown in FIG. 2 of the present disclosure. In some such examples, as shown blood is received into the defoaming device 200 via one or more pumps 100 that pressurize the blood 50 and convert it into pressurized blood 51 which then passes through injectors 150 that ozonate the pressurized blood 51 under pressure to form foamed ozonated blood 52. For example, ozonation is done under a pressure of around 1 bar. In the example shown in FIG. 1, there is one pump 100 is shown. Thus, the one or more pumps 100 aid in pressurizing said blood 50, resulting in the foamed ozonated blood 52.

In some such examples, a peristaltic pump 160 is used that pumps blood 50 into the blood foam management system 1000 of the present disclosure. In some embodiments, the blood is cooled to about 3 degrees Celsius and passes towards the injectors 150 where the ozone gas 64 is connected and injected into the blood, (or in other words where the intake of ozone gas 64 is). Thus, in some embodiments of the present disclosure, there is a cooling exchange at the intake or inlet of blood, i.e. when the blood is received from the patient's body. Similarly, in some such embodiments, there is also a heat exchange at the outlet, at the point where the ozonated blood is returned to the patient's body, preferably at room temperature. In some examples the injectors 150 inject microbubbles of ozone containing gas into a flow of blood under pressure, thereby creating foam and thus pressurized foamed ozonated blood 52. In some such examples the microbubbles have a mean diameter of less than about 5 µm. Thus, microbubbles hat create and/or form the foam, also have a mean diameter of less than about 5 µm. In a particular example, the microbubbles have a size of about 2 µm. Thus, microbubbles that form the foam also have a mean diameter of about 2 µm. In some such examples the injected microbubbles are entrained the flow of blood and/or are dissolved in the blood. In one such example the microbubbles are substantially dissolved in the blood.

In some such embodiments, the pressure and/or the flow rate are controlled. In some such embodiments, the flow rate and/or pressure controlled and/or exerted by the one or more pumps 100 and/or the one or more injectors is such that the microbubbles substantially completely dissolve in the flow of blood and have a diameter of less than about 5 µm, preferably about 2 µm. In some such embodiments, controlling the flow and/or pressure of the ozone containing gas and/or blood flow rate and/or pressure by the system of the present invention, may prevent the microbubbles from coalescing into larger bubbles, such that essentially substantially all of the microbubbles entrained int the blood flow, have a diameter of less than about 5 µm. In some such cases, the microbubbles that have a diameter of less than about 5 µm prevent damage to the blood cells during ozonation thereof. In such embodiments, the microbubbles form foam, and said foam is prevented from coalescing into larger bubbles because of the controlled pressure and/or the flow rate. Thus, in some embodiments of the present disclosure, the foam is comprised of microbubbles as described herein.ln some such examples, said microbubbles are dissolved substantially in said blood.

With reference again to FIG. 1, the blood 50 from the patient is received and pumped using the peristaltic pump 160, and the pressurized blood 51 travels in a substantially straight line towards the injectors 150, for example vertically upwards. other words, moves up in the defoaming device 200. Foam 60 is generated at the injectors 150 and foam 60 is created or received in the evaporator or defoaming device 200. The pressure of the ozone gas 64 moves the foam 60 upwards from the evaporator or defoaming device 200. Thus, pressure in the blood 50 is generated by both the peristaltic pump 160 as well as the ozone injectors 150 operating at a pressure of up to about 1 bar, for example. In another example, the pressure in the blood 50 is generated by both the peristaltic pump 160 as well as the ozone injectors 150 operating at a pressure of up to about 1.5 bars.

Additionally, in accordance with some embodiments of the present disclosure, even though ozone is highly oxidizing, as may be known in the art, the present inventors have discovered that that blood 50 is not destroyed through ozonation under repeated ozonation of blood 50 for about 8 to about 10 hours, in live animal trials, specifically of porcine blood. Thus, the present inventors have discovered that repeated ozonation does not damage the blood cells and is expected to be just as safe in humans.

In accordance with a system 1000, for managing blood foam in accordance with embodiments described herein below, a lot of different pressures can be used in the machine or in other words, the system 1000. For example, if you alter or change the pressurization of the blood 50, you can change the size of the ozone bubbles. The present inventors have discovered that in present systems or procedures for ozonation, ozonation can result in thick or heavy foam 60. The foam 60 once it is collected in the collection chamber or chamber or defoaming chamber 202 of the defoaming device or evaporator 200, the system 1000 of the present disclosure, allows the foam 60 to disappear the entire time the system 1000 is operating. In other words, some embodiments of the present disclosure provide for continuous removal of foam from the foamed oxygenated blood 52 using the defoaming device or evaporator 200.

### Defoaming

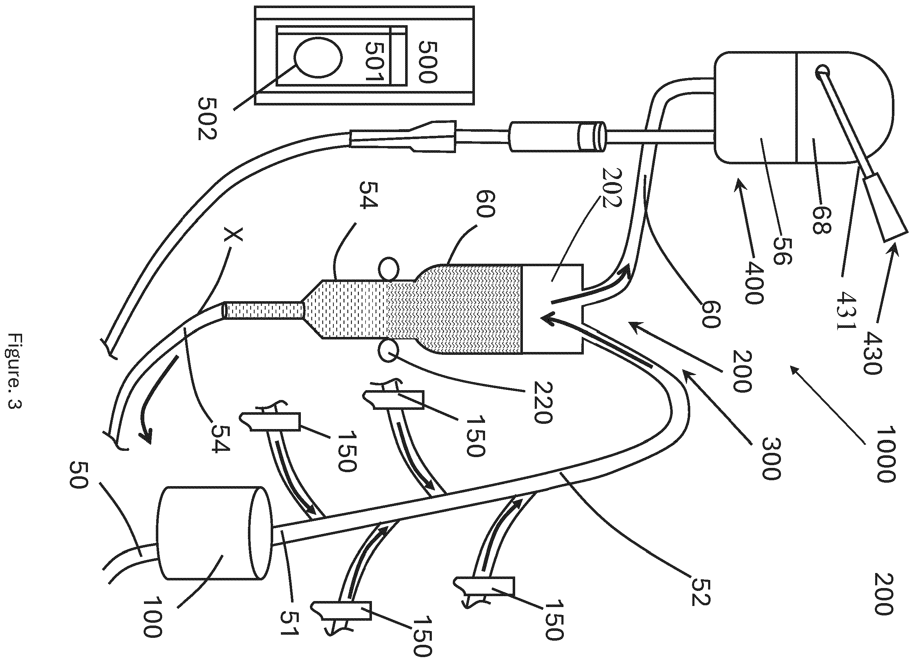

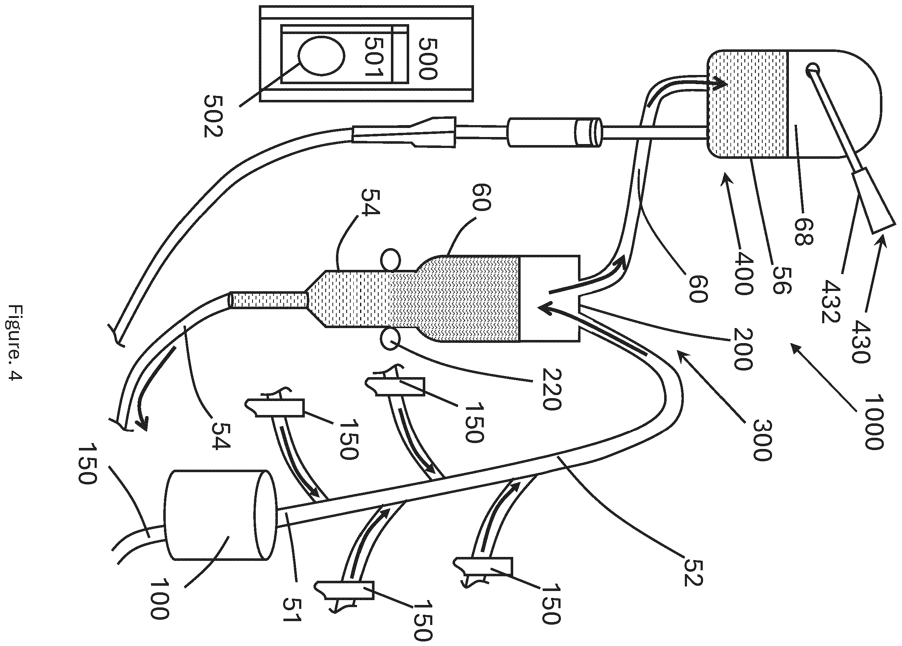

In some examples, the injection of ozone 64 by the injectors 150 is substantially vertically upward, and the zone gas and blood (i.e. blood with the substantially dissolved ozone) then goes into the evaporator or the defoaming device 200. In some examples, this may also travel substantially vertically upwards within the defoaming device 200. In some instances, a lot of or a substantial amount of foam is collected or received in the evaporator or the defoaming device 200. As 10% gas is connected to the blood 51, this substantial amount of foam 60 comes up or upwards in the defoaming device 200. The defoaming device 200 is configured for receiving the pressurized foamed ozonated blood 52 for separating the received blood into foam 60 and ozonated blood 54 inside the chamber 202 of the defoaming device 200, as illustrated in FIG. 3, thus defoaming the foamed ozonated blood 52. Thus, in some embodiments of the present disclosure, the defoaming device facilitates processing of the pressurized foamed blood received in a chamber 202 of the defoaming device 200. The foam 60, as it moves upwards in the chamber 202 under pressure, separates from the ozonated blood 54, as illustrated in FIG. 3 and FIG. 4. The defoaming device 200 facilitates return of ozonated blood 54 that has been collected from the foamed ozonated blood 52, and returned back to a patient's body, substantially without the foam, as illustrated in one example in FIG. 3. The goal of the embodiments of the present disclosure is to not to discard the foam 60, unlike conventional procedures where the foam 60 is discarded after the procedure, but rather to utilize foam 60 for the benefit of the patient. Since the foamed ozonated blood 52 received into defoaming device 200 is pressurized, the foam 60 that is separated from the ozonated blood 54 in the chamber 202 of the defoaming device 200 is also pressurized.

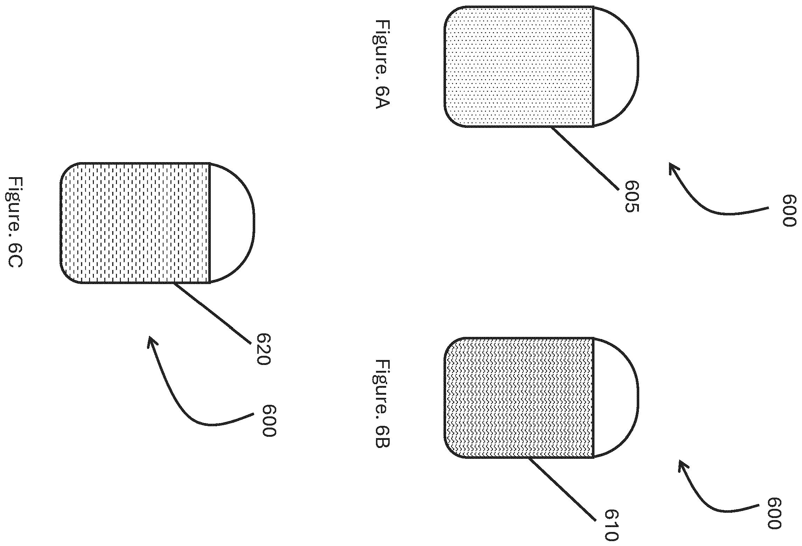

Thus, in some embodiments of the disclosure, the foamed ozonated blood 52 is received directly from a patient, wherein primary ozonated blood 54 is returned to the patient in real time (online), while the foam management apparatus 300 is connected to the patient and the foam 60 has been removed therefrom. Alternatively, the foamed ozonated blood 52 may not be directly received from the patient, but rather may be taken from the patient and stored in a blood bag. This foamed ozonated blood 52 from the blood bag may then be fed into the defoaming device 200 of the foam management apparatus of the present disclosure. Thus, in alternative embodiments of the present disclosure, the foamed ozonated blood 52 is received from an intermediate blood bag 610, as shown in FIG. 6B, wherein after processing the foamed ozonated blood using the apparatus/systems/methods of the present disclosure, the ozonated blood 54 returned to a second blood bag 620, with reference to FIG. 6C, to be returned to the patient, the foam management apparatus 300 being disconnected from the patient. Thus, in this case, in other words, the procedure is done offline. In both instances the foam 60 has been removed by the defoaming device 200. Such materials include materials typically used to transfer or store blood, such as those used in tubing for transfusions.

In some embodiments of the present disclosure, the defoaming device 200 comprises a sensor 220 that measures the level of ozonated blood 54 in the defoaming device 200 and provides a level control. It essentially serves as a pressure gauge. If the level of the ozonated blood 54 in the defoaming device 200 is under substantially the same pressure over a period of time, then the level of ozonated blood 54 detected within the defoaming device 200 is also essentially the same. Thus, the level of ozonated blood 54 in the chamber 202 of the defoaming device 200 functions as an indicator of pressure. Thus, the sensor 220 provides feedback regarding the pressure in the defoaming device 200. The sensor 220 is a part of and/or is located in and/or adjacent, the defoaming device 200, with reference now to FIG. 3 and FIG. 4. This sensor 220 checks if the defoaming device 200 is working under optimal pressure. If there is mixing observed in between the foam 60 and the ozonated blood 54, the pressure can be increased, thus raising the level of ozonated blood 54 in the chamber 202 of the defoaming device. This can be done by raising the pressure of the one or more pumps 100 and/or the pressure at which ozonation occurs at the injectors 150. In some such examples, the sensor 200 may comprise one or more sensors 220. In one example, the one or more sensors comprise optical sensors.

### Antifoaming

With reference now to FIG.4, in some such embodiments, the pressurized foam 60 exits the defoaming device 200 and enters the antifoaming device 400 which is a part of the foam management apparatus 300, where it processes the foam 60 into ozonated blood, as outlined further in detail herein below. Thus, the foam management apparatus 300 allows processing of said pressurized foam 60 for antifoaming, to retrieve remaining ozonated blood therefrom for return to the body. As outlined herein above, the foam 60 is collected in the evaporator or the defoaming device 200. The pressure of the ozone gas 64 in the pressurized blood 51 that has been pressurized by a pump 100 such as a peristaltic pump 160, that moves the foam 60 upwards. The foam 60 exits the evaporator or defoaming device 200 and enters the antifoaming device 400, which in some examples comprises a bag or pouch, as shown, with reference to Fig. 4, where the foam 60 is converted into liquid, i.e. back into blood, which is ozonated blood. Further details are described herein below.

In some such embodiments, the pressurized foam 60 is converted into a secondary ozonated blood 56. The secondary ozonated blood 56 that is recovered from the foam 60 is more ozonated than ozonated blood 54, as it is inundated with microbubbles of ozone which take the appearance of foam 60 when mixed with blood. Thus, when the foam 60 is processed to allow egress of gas from the foam 60, the foam is converted into secondary ozonated blood 56. In other words, the foam 60 is degassed. In some instances, this can be directly returned to the body, substantially without the foam 60. In other instances of the embodiments of the present disclosure, it can be stored in a blood bag and given to the patient when the need arises.

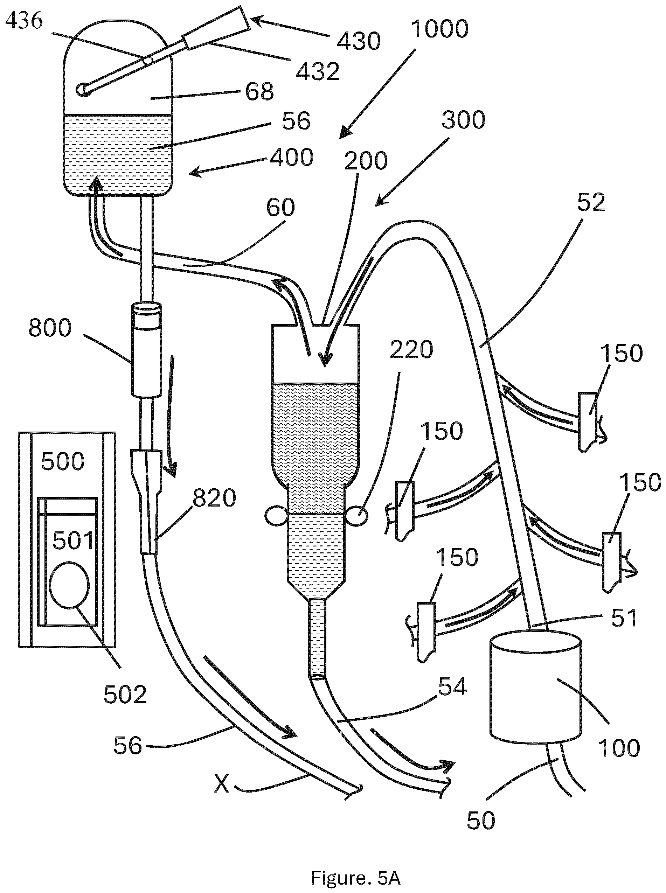

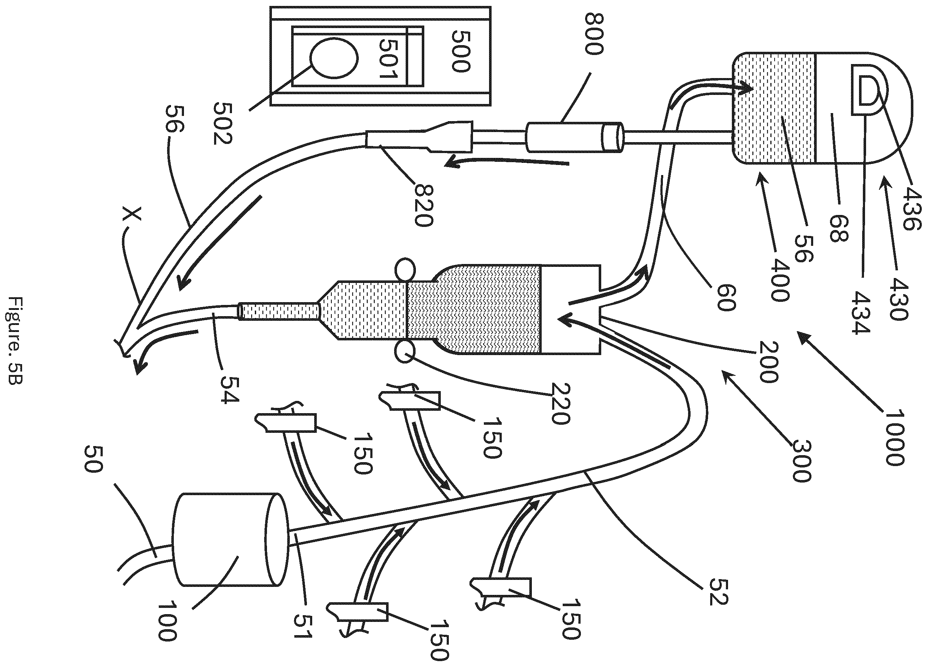

The details of the antifoaming process are described further with reference to FIG. 4. The foam management apparatus 300 of the present disclosure is provided, that comprises an antifoaming device 400. The antifoaming device 400 is positioned above the defoaming device 200, for receiving said pressurized foam 60. Since the foam 60 is pressurized, it can travel against gravity into the antifoaming device 400 said antifoaming device 400 that is position higher than the defoaming device 200. The antifoaming device 400, comprising an egress 430, or in other words an outlet and/or air vent 430, to allow a gas 68 to be separated from said foam 60, allowing said foam 60 to be converted into the secondary ozonated blood 56. In some such examples, the antifoaming device 400 comprising an antifoaming bag 400. In other words, the foam 60 is degassed to allow gases or vapor 68 to separate from the liquid or in other words the secondary ozonated blood 56, as shown in FIG. 5. The antifoaming device 400 then allows the gas 68 to leave from the egress or aperture 430, this allows the foam 60 to be converted into secondary ozonated blood 56. Since the antifoaming device 400 is positioned higher than the defoaming device 200, it allows gravity to aid in moving the secondary ozonated blood 56 to be returned to the patient via a line or tubing X.

In one particular example, as shown in Figs. 1 to 4 and 5A, the egress 430 comprises a needle 431 having a shunt or aperture 432. In other words, the needle 431 has a hole or aperture 432 that is pointing upwards to allow the gas or air to escape as the gas or air is also under pressure. In some such embodiments, the hole or aperture 432 allows the pressure to be relieved from the system 1000 of the present disclosure, for example, from the pressurized blood product such as blood 50 flowing through the system 1000. In other examples, as shown in Fig. 5A, the egress 430 may comprise a window 434, as shown in Fig. 5B. In some examples, the egress 430 contains a valve 436, such as a one-way valve, to allow air to escape while substantially preventing entry of any foreign material through the egress 430. In some such embodiments, the valve 436 is a check valve for allowing the air to exit. Thus, in some embodiments the needle 431 contains a valve 436.

The recovery of secondary ozonated blood 56 also facilitates in treatment of the patient as it allows conversion of foam 60 into secondary ozonated blood 56 that is returned to the patient, either via blood bag via an IV tube, or directly from the antifoaming device 60 as part of live procedure, as shown in FIG. 5A and FIG. 5B. As the antifoaming device 60 is positioned above the defoaming device 200. This secondary ozonated blood 56 results in better ozonation treatment for the patient, for example for effective treatment of sepsis, as it is more ozonated than the primary ozonated blood 54. Thus, the secondary ozonated blood 56 combined with the ozonated blood 54 results in more effective treatment of pathogens in the blood, such as bacteria, viruses or others, resulting in more effective treatment of sepsis, for example. This may allow for faster recovery time Also, since the foam 60 isn't discarded, unlike conventional approaches, and is converted to secondary ozonated blood 56, it allows substantially all of the blood that is removed from the patient in a live ozonation procedure, to be returned back to the patient. Thus, it results in no or minimal loss of blood due to the procedure. This may allow for faster recovery time. Unlike conventional treatments, where foam may be discarded which may result in removing too much blood from the patient and placing the patient under medical stress, the present disclosure allows substantially all of the blood that is removed from the patient to be returned back to the patient. This removes the added stress on the patient due to the removal of blood and may help aid in a faster recovery, as substantially all of the blood volume taken from the patient for ozonation, is returned to the patient as ozonized blood.

### Alternative antifoaming devices

In alternative embodiments of the disclosure as described herein, an alternative antifoaming device may be used that is compatible with the foam management apparatus, system and methods of the present disclosure as described in the present application. In some embodiments the antifoaming device 400, may not comprise an egress, and may define an enclosed volume for receiving the foam, where the enclosed volume allows the gas to dissipate, leaving blood product behind, that substantially does not contain gas. In other words, the enclosed volume is sized sufficiently to accommodate the gas as it dissipates, sufficiently to allow the liquid such as blood product to be substantially free from dissolved gas and thus foam. The antifoaming device 400 can be used in all of the medical procedures and/or applications described in the present disclosure. In some such examples, the enclosed volume allows the gas to dissipate leaving behind ozonated blood that can be returned to the patient, where the ozonated blood substantially does not contain gas.

### Other Applications

In some such embodiments of the present disclosure, the foam management apparatus comprises a defoaming device 200 that receives a foamed blood product, where a chamber 202 of the defoaming device 200, separates the foamed blood product into foam and the blood product, where the blood product can be returned to the patient, substantially without the foam.

In some embodiments of the present disclosure of the foam management apparatus, the antifoaming device 400 can be used independently form the defoaming device 200 and may be used for other applications where foam 60 is required to be removed from a blood product in conventional procedures. In these embodiments of the disclosure, the antifoaming device 400, can be used to convert foam 60 into blood as may be required. For example, under use of a heart-lung machine, the antifoaming device 400 of the present disclosure can be used to convert foam 60 created during oxygenation of the blood to be received from the heart-lung machine and converted into oxygenated blood for the benefit of the patient. In some such embodiments, the antifoaming device 400 is configured to remove foam comprising of microbubbles, where the microbubbles are substantially dissolved in the blood product such as blood, in accordance with embodiments of the present disclosure. In some examples, the microbubbles are substantially completely dissolved in the blood product.

In some such cases, the foam management apparatus 300 comprises an antifoaming device 400 for receiving a foam from a foamed blood product, said antifoaming device comprising an egress 430 to allow a gas 68 to be separated from said foam 60, allowing said foam 60 to be converted into said secondary recovered blood product, wherein said antifoaming device converts said foam back into said blood product. In some examples of this disclosure, the foam management apparatus 300, further comprises a defoaming device 200 for receiving a foamed blood product for separating a foam 60 therefrom for processing, where the foam is fed into the antifoaming device 400 and the primary recovered blood product is returned to the patient. In one such example, the foamed blood product may be foamed oxygenated blood form the heart-lung machine and the primary recovered blood product may be oxygenated blood that is recovered from the defoaming device 200 and returned to the patient substantially without the foam. Additionally, the foam is converted back into oxygenated blood, where the secondary recovered blood product is oxygenated blood recovered from the foam. This may be more oxygenated than the primary recovered blood product and thus may provide benefits similar to those described herein above. It may increase the amount of oxygenated blood returned to the patient as blood loss is minimized as foam is not discarded. Additionally, it provides increased oxygenation to the patient as discussed above.

In some examples of this disclosure, the foam management apparatus 300, further comprises a defoaming device 200 comprising a chamber 202 for receiving a foamed blood product comprising pressurized foamed ozonated blood 52 for separating a foam 60 therefrom for processing, to enable return of the blood product comprising primary ozonated blood 54 from said foamed ozonated blood 52, to the body, said foam 60 being pressurized. The foam management apparatus 300 further comprises an antifoaming device 400 for receiving said pressurized foam 60, said antifoaming device 400, comprising an egress 430 to allow a gas 68 to be separated from said foam 60, allowing said foam 60 to be converted into the blood product comprising secondary ozonated blood 56, the secondary ozonated blood 56 being more ozonated than ozonated blood 54. In such embodiments, said blood product comprising said primary ozonated blood and said secondary ozonated blood, is returned to the body substantially without the foam.

In some embodiments of the present disclosure, a foam management apparatus 300 is disclosed for a foamed blood product comprising: an antifoaming device 400 for receiving a foam from a foamed blood product, said antifoaming device comprising an egress 430 to allow a gas 68 to be separated from said foam 60, allowing said foam 60 to be converted into said blood product; wherein said antifoaming device converts said foam back into said blood product. In some such embodiments, the antifoaming device converts the foam comprising of microbubbles, back into the blood product. In some such embodiments, removal of gas 68 is a passive process guided by the pressures in the system, due to pressurized blood product in the system in accordance with embodiments of the present disclosure.

### Systems

Systems as disclosed and described herein, in accordance with some embodiments of the present disclosure, with reference to drawings enclosed herewith:
With reference now to Fig. 1, in some broad examples of the present disclosure, a foam management system 1000 is disclosed for managing foam during ozonation, comprising the foam management apparatus 300 as described herein above. The system 1000 comprises one or more pumps 100 that allow blood 50 leaving the patient to be pumped towards the foam management apparatus 300. The one or more pumps creates a pressurized system which places the blood 50 under pressure forming pressurized blood 51, allowing the pressurized blood 51 to move through the system, and as such, the foam management apparatus 300 of the present disclosure. The system additionally comprises one or more injectors 150, that are placed downstream from one or more pumps 100 as shown in Fig. 1, for injecting pressurized ozone into the blood 50, forming foamed pressurized blood 52. Once the blood 50 coming from the patient has passed through the one or more pumps and/or the one or more injectors it is converted into pressurized foamed ozonated blood 52. The ozonated blood is desirable for the patient, however, the foam in the ozonated blood is dangerous if returned to the patient as it can cause pulmonary embolism which can lead to cardiac arrest. As such, the foam needs to be removed from the foamed ozonated blood 52 before the ozonated blood can be returned to the patient, to treat pathogens in the blood such as virus, bacteria and/or others that can cause sepsis.

### Defoaming

As such, the foam management system 1000 comprises a defoaming device 200 for receiving and processing the pressurized foamed ozonated blood 52, as shown in FIG.2. The defoaming device 200 comprises a chamber 202 for separating foam 60 from the ozonated blood 54. Once the two components have been separated by and/or in the chamber 202, they are separated into foam 60, and primary ozonated blood 54 which can then be returned to the patient, as shown in FIG.3. The foam 60 that is removed is still pressurized. In such embodiments, a sensor 220, with reference again to FIG. 3, can be provided in said defoaming device 200 for checking the level of blood in the defoaming device 200, wherein if the level of ozonated blood 54 is too low, hence permitting mixing of foam 60 and ozonated blood 54, the pressure in the one or more pumps 100 and/or the injectors 150 can be increased. This will allow the foam 60 to separate from the ozonated blood 54 more cleanly and efficiently. In some embodiments of the present disclosure, the pressure exerted by the one or more pumps 100 and/or injectors is about 1.5 bars. In some such cases, this provides the advantage of preventing damage to the blood cells. In some such examples of a foam management system and apparatus, the foam 60 comprises microbubbles of ozone containing gas, wherein said microbubbles are dissolved substantially in said foamed ozonated blood 52.

### Antifoaming

Unlike conventional procedures where foam 60 is taken and discarded, the system of the present disclosure allows the foam 60 to be recycled back into usable ozonated blood. The foam management apparatus 300 allows processing of the pressurized foam 60 to retrieve the remaining ozonated blood from the foam 60 so it can be returned to the patient. More specifically, the pressurized foam 60 is converted into a secondary ozonated blood 56, and this secondary ozonated blood 56 is more ozonated (or more ozonated) than ozonated blood 54. This makes the treatment more effective as more ozonated blood is returned to the patient. Therefore, in accordance with the present disclosure primary ozonated blood 54 and said secondary ozonated blood 56 returned to the patient substantially without the foam. In such embodiments, the system 1000 further comprises an antifoaming device 400 for receiving said pressurized foam 60, as shown in FIG. 4, said antifoaming device 400, comprising an egress 430 to allow a gas 68 to be separated from said foam 60, allowing said foam 60 to be converted into a secondary ozonated blood 56, said secondary ozonated blood 56 being more and/or highly ozonated than ozonated blood 54. As shown in FIG.5A and Fig. 5B, this more ozonated secondary ozonated blood 56) is returned to the patient. The antifoaming device thus converts foam 60 back into ozonated blood, specifically secondary ozonated blood 56, thus utilizing foam instead of discarding it like conventional procedures. In some such embodiments, the foam 60 comprises microbubbles of ozone containing gas, wherein said microbubbles are dissolved substantially in said foamed ozonated blood 52. Thus, the foam 60 formed from the microbubbles is converted back into ozonated blood, using the antifoaming device of the present invention. Converting foam 60 into ozonated blood allows both the advantage of retrieving more ozonated blood for the patient but also allows the substantially all of the blood volume received from the patient to be returned to the patient. Both these factors, either individually or in combination help procedural efficacy in treating conditions such as sepsis with increased ozonation of returned blood, and also prevent blood loss due to the procedure, aiding in further recovery of the patient.

In some embodiments of the present disclosure, the tubing X that takes the secondary ozonated blood 56 back to the patient comprises IV drip chamber 800 and/or an IV clamp or pinch 820, with reference to Figs. 5A and 5B. The IV drip chamber or in other words a drip bulb, allows gas (such as air) to rise out from a fluid, such as blood 50, in the present disclosure, so that it is not passed downstream towards the patient's body. It is employed in the delivery system of ozone intravenous therapy of the present disclosure, as outlined herein, and acts to prevent air embolism.

In some embodiments of the present disclosure, the IV drip chamber 800 may be used in conjunction with an IV claim 820 also shown in Figs. 5A and 5B, on the line or tubing X, that is taking the secondary ozonated blood 56 back to patient's body. In some examples, the IV claim 820 may be a roller clamp. The IV clamp functions to control the flow rate of the secondary ozonated blood 56 that is being returned to the patient. In embodiments where the IV clamp 820 comprises a roller IV clamp, a roller of the roller IV clamp can be rolled up to increase the flow rate of the secondary ozonated blood 56 back to the body. Alternatively, the roller of the roller IV clamp can be rolled down, to decrease the flow rate of the secondary ozonated blood 56 going back to the patient's body. Even though in the embodiments shown in the drawings the IV drip chamber 800 and/or an IV clamp or pinch 820, are shown with reference to tubing X carrying secondary ozonated blood 56 to the body, shown in Figs. 5A and 5B, the IV drip chamber 800 and/or the IV clamp or pinch 820 may also be positioned on the tubing X, carrying primary ozonated blood 54 to the patient's body with reference to FIG. 3, for example. Positioning the drip chamber 800 and/or IV clamp 820 at this point, also provides the same advantages as noted herein above. In other words, the IV drip chamber 800 and the IV clamp 820 may be positioned at points where the system 1000 returns the ozonated blood 54, 56 back to a patient's body.

With reference again to FIGS. 1-4 and FIG. 5A, the two blood lines are not joined. In other words, the tubing or line X taking the primary ozonated blood 54 back to the body (FIG. 3), does not join or mate with the tubing X, taking the secondary ozonated blood 56 (FIG. 5A) back to the body. In alternative embodiments, the tubing or line X taking the primary ozonated blood 54 back to the body (FIG.3) is joint to or mates with tubing or line X taking the secondary ozonated blood 56 back to the body (FIG. 5B).

In some embodiments of the present disclosure, a flow control valve may be provided at the input or intake lines (that bring blood 50 from the body) into the system 1000 and/or the output or outtake lines (that take ozonated blood 54,56 back to the body), which may assist in controlling the rate of inflow of blood 50 into the system 1000 and/or in controlling the rate of outflow of ozonated blood 54, 56 from the system 1000 to the patient's body.

In some embodiments of the system of the present disclosure, a foam management system 1000 is provided where the system comprises the foam management apparatus 300 as described herein and one or more processing units 500 to operate said one or more of, the one or more pumps 100, the one or more injectors 150, the defoaming device 200, the antifoaming device 400, and/or the sensor 220, thereby separating said foam 60 from said blood product and converting said foam back into said blood product, allowing substantially all of the blood product to be recovered. In some such embodiments the foam comprises of microbubbles. In some examples, microbubbles are substantially dissolved in said blood product, the microbubbles being sized as provided herein in the present disclosure. In some examples, the microbubbles are substantially completely dissolved in said blood product.

In some such embodiments, said one or more processing units 500 allow for automated system for control of one or more of: the foam management apparatus 300, the one or more pumps 100, the one or more injectors 150, the defoaming device 200, the antifoaming device 400, and/or the sensor 220, and/or other components of the system, thereby facilitating managing of foam to allow ease of operation by the operator as well as allowing for automatically adjusting defoaming and antifoaming to optimize return of blood product back to the patient, substantially without the foam. The automation/automated control, may offer the advantage of ease of use for the operator and may make the system and thus the procedure of ozonating blood for example to treat sepsis, more repeatable and predictable. This may additionally make the procedure safer for the patient.

### Other applications the System

As outlined herein above, in some embodiments of the present disclosure the antifoaming device 400 can be used independently form the defoaming device 200 and may be used for other applications where foam 60 is required to be removed in conventional procedures. In these embodiments of the disclosure, the antifoaming device 400, can be used to convert foam 60 into blood as may be required. For example, under use of a heart-lung machine, the antifoaming device 400 of the present disclosure can be used to convert foam 60 received from the heart-lung machine, into oxygenated blood for the benefit of the patient. In some such embodiments, a system 1000 is disclosed for removing foam from a blood product. The system comprises one or more pumps 100, for pressurizing the blood product creating a foamed blood product (these pumps can also include systems for adding oxygenation or ozonation to the blood product, including other applications). The system then comprises a foam management apparatus 300 comprising a defoaming device 200 for receiving the pressurized foamed blood product for separating a foam 60 therefrom to return a primary blood product recovered from said foam removal to the patient. The foam 60 being pressurized and thus may remain pressurized. The foam management apparatus 300 additionally comprising an antifoaming device whereby said pressurized foam 60 is converted into the blood product as secondary recovered blood product, and wherein said primary and secondary blood product is returned to the patient substantially without the foam. For example, the antifoaming device 400 can be used to convert foam to oxygenated blood in a heart lung machine where foamed oxygenated blood is present due to the oxygenation. In some such embodiments where the antifoaming device 400 converts foam into the blood product, such as into oxygenated blood, the foam comprises substantially of microbubbles. In some such examples, the microbubbles are substantially dissolved in said oxygenated blood.

### Ozonation Application of the System

As outlined herein above, such a foam management system of the present disclosure that allows for foam to be converted back into the blood product itself can also be used for blood products that have been ozonated. Such a system of the present disclosure comprises one or more pumps 100, for pressurizing a blood product, wherein the blood product comprises blood 50, into pressurized blood 51. As shown in FIG. 1, the system also comprises one or more injectors 150, for injecting pressurized ozone into the blood 50, forming foamed pressurized blood 52, where the one or more injectors 150 and/or said one or more pumps 100 create a pressurized foamed ozonated blood 52. As described previously, the system further comprises a foam management apparatus 300 comprising a defoaming device 200 for receiving the foamed blood product comprising the pressurized foamed ozonated blood 52 for separating a foam 60 therefrom for processing, to enable return of ozonated blood 54 from said foamed ozonated blood 52 to a patient, as shown in FIG. 2 and FIG. 3. The foam 60 is pressurized. The chamber 202 of the defoaming device 200 separates the foam 60 from the ozonated blood 54. The foam management apparatus 300 comprises an antifoaming device 400 that allows processing of said pressurized foam 60 to retrieve remaining blood product comprising the ozonated blood therefrom for return to the patient. With reference to FIG. 4 and FIG. 5 said antifoaming device 400, comprising an egress 430 to allow a gas 68 to be separated from said foam 60, allowing said pressurized foam 60 to be converted into the blood product comprising a secondary ozonated blood 56, said secondary ozonated blood 56 being more ozonated than ozonated blood 54. Thus, secondary ozonated blood 56 has a greater content of ozone and/or ozonation than primary ozonated blood 54. In such examples, wherein the ozonated blood 54 and said secondary ozonated blood 56 are to be returned to the patient substantially without the foam.

In some such embodiments of the system 1000, wherein the foam management system comprises one or more processing units 500, as shown in FIGS.1 to 4 and FIGS. 5A, 5B, to operate said one or more of: the one or more pumps 100, the one or more injectors 150, the defoaming device 200, and the antifoaming device 400, and the sensor 220. In some such examples, the one or more pumps 100, injectors 150, the antifoaming device 400, and the sensor 220 may be automated, facilitating the procedure. This may provide the advantage of reducing time for the procedure and facilitating the operator in running the procedure. In some such examples, the one or more injectors 150, comprise at least four injectors 150. In another example, the one or more injectors 150 for injecting pressurized ozone into the blood 50 are operating at a pressure of 1 bar, with reference to FIG. 1. In another example, said one or more pumps comprises a tube pump or a peristaltic pump 160. In some embodiments of the system of the present disclosure the foam management apparatus 300 comprises the foam management apparatus 300 as disclosed herein above.

In a specific example as outlined above, the blood 50 can be received directly from a patient, and the ozonated blood 54 and secondary ozonated blood 56, can be both returned to the patient in real time, while the system 1000 is connected to the patient using one or more blood bags 600. In other examples, alternatively, the blood 50 is received from a first blood bag 605, for example from a blood bank, as shown in FIG. 6A, and after processing said blood 50 using the apparatus, system and/or methods of the present disclosure, the ozonated blood 54 and the secondary ozonated blood 56, are both returned to another blood bag 600 (which may be a different blood bag each or may be the same blood bag 620), as shown in FIG. 6C, to be returned to a patient while the system 1000 is disconnected from the patient.

In some embodiments of the present disclosure, a foam management system 1000 is disclosed for managing a foamed blood product, the system comprising: one or more pumps 100, for pressurizing the blood product creating a foamed blood product; a foam management apparatus 300 comprising an antifoaming device

A foam management system 1000 for managing a foamed blood product, the system comprising: one or more pumps 100, for pressurizing the blood product creating a foamed blood product; and a foam management apparatus 300 comprising an antifoaming device 400 having an egress 430, wherein a pressurized foam 60 from said foamed blood product is separated into gas 68 and secondary recovered blood product, said gas 68 leaving through said egress 430, wherein said foam 60 is converted into the blood product as a secondary recovered blood product, that is returned to the patient substantially without the foam.

In some embodiments of the above example, a foam management system 1000 is disclosed for managing a foamed blood product, the system comprising: one or more pumps 100, for pressurizing the blood product creating a foamed blood product; a foam management apparatus 300 comprising a defoaming device 200 for receiving the pressurized foamed blood product for separating a foam 60 therefrom to return a primary blood product recovered from said foam removal to the patient, said foam 60 being pressurized; the foam management apparatus 300 comprising an antifoaming device whereby said pressurized foam 60 is converted into the blood product as secondary recovered blood product; and wherein said primary and secondary blood product is returned to the patient substantially without the foam. In some such examples one or more pumps 100 may comprise a heart-lung machine for oxygenation of blood and/or other medical devices that result in foaming of blood, such as an extracorporeal blood circulation device such as an apheresis machine, dialysis machine, or a heart and lung machine. In some such embodiments, foam comprises microbubbles as outlined in the present disclosure as described herein.

### Methods

Methods as disclosed and described herein, in accordance with some embodiments of the present disclosure, with reference to drawings enclosed herewith:

### Defoaming and Antifoaming

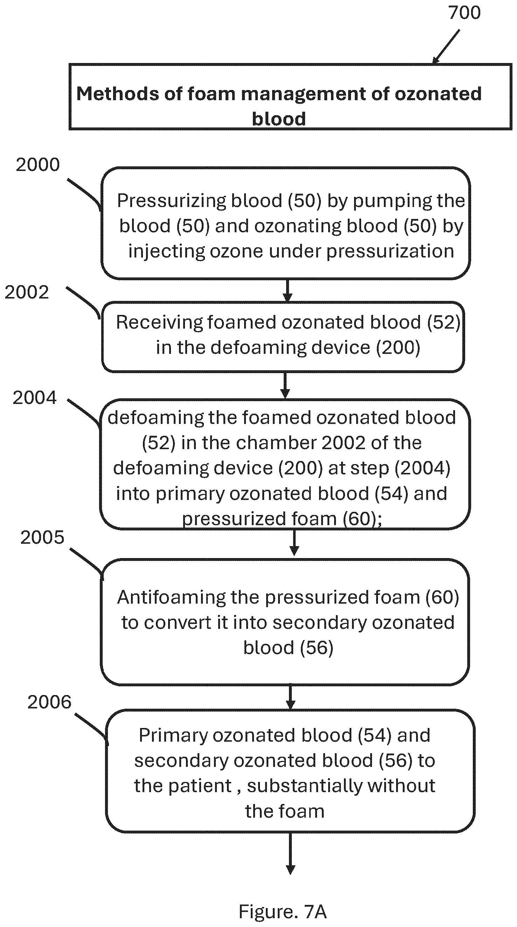

A method for managing foam 700 using a foam management apparatus 300 comprising a defoaming device 200 and an antifoaming device 400, the method as outlined in FIG 7A. The method comprises the steps of: (i) pressurizing blood 50 by pumping the blood 50, at first step 2000 into pressurized blood 51, (ii) ozonating blood 50 by injecting ozone under pressurization, at first step 2000 into foamed ozonated blood 52, (iii) receiving foamed ozonated blood 52 into the defoaming device 200, at step 2002, (iv) defoaming the foamed ozonated blood 52 in the chamber 202 of the defoaming device 200 at step 2004 into primary ozonated blood 54 and pressurized foam 60, (v) antifoaming the pressurized foam 60 for processing said pressurized foam 60 to convert it into secondary ozonated blood 56, the secondary ozonated blood 56 being more ozonated and/or highly ozonated than primary ozonated blood 54, at step 2005. In other words, secondary ozonated blood 56 has a higher content of ozone than primary ozonated blood 54; and returning ozonated blood 54 and the secondary ozonated blood 56 to the patient, at step 2006, substantially without the foam 60.

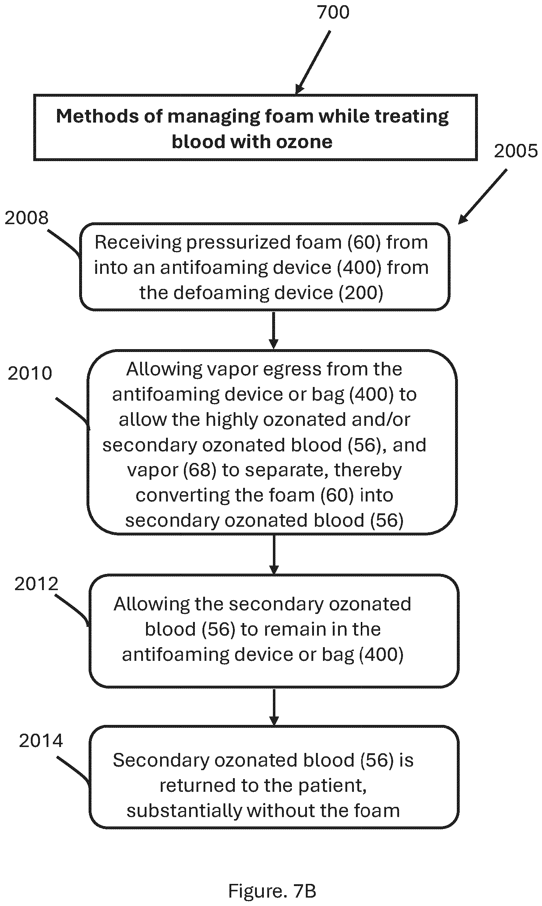

As shown in FIG. 7B, at step 2005, the method of managing foam 700 further comprises the steps of: (i) receiving pressurized foam 60 into an antifoaming device 400 from the defoaming device 200 at step 2008, (ii) permitting gas egress from the antifoaming device 400, which in some instances of the present disclosure comprises an antifoaming bag as shown in FIGS. 1 to 4, Fig. 5A and Fig. 5B, which allow the secondary ozonated blood 56 and gas and/or air 68 to separate, as shown in step 2010 (iii) wherein the secondary ozonated blood 56 remains in the antifoaming device 400, at step 2012; and returning the secondary ozonated blood 56 back to the patient, substantially without the foam. As outlined in the present disclosure herein above, the secondary ozonated blood 56 is more ozonated than primary ozonated blood 54 and when it is returned to the patient, it increases procedural efficacy in terms of treating sepsis and/or similar conditions. Additionally, since the entire blood volume of the patient is returned to the patient, as foam 60 isn't discarded, the patient does not experience blood loss due to the procedure and this may aid in a faster recovery.

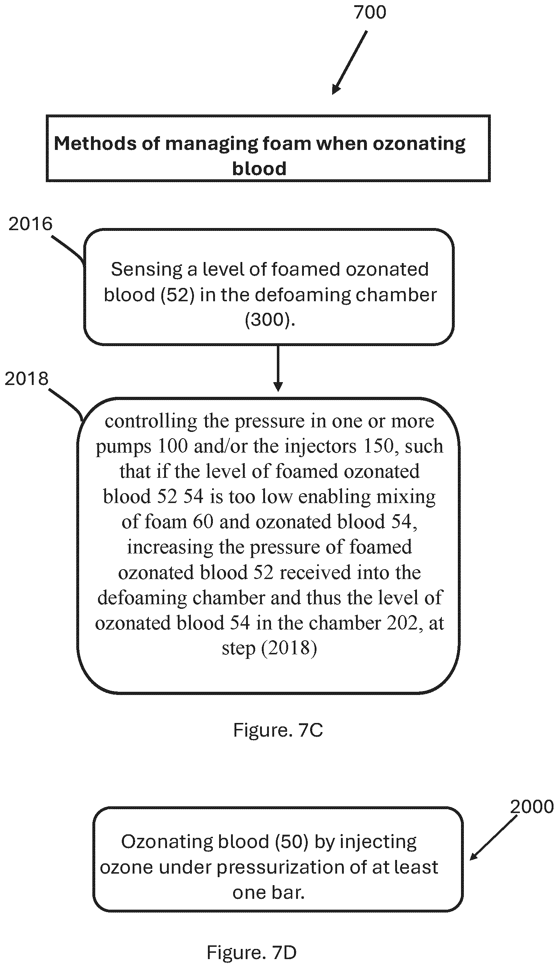

As outlined herein above as well, as shown in FIG. 7C, additionally a method 700 of the present disclosure for managing foam comprises the steps of: (i) sensing a level of ozonated blood 52 in the defoaming device 200, at step 2016; and (ii) controlling the pressure in one or more pumps 100 and/or the injectors 150, such that if the level of ozonated blood 54 is too low enabling mixing of foam 60 and ozonated blood 54, increasing the pressure of foamed ozonated blood 52 received into the defoaming chamber 202 of the defoaming device 200, at step 2018. This allows the defoaming device 200 to work optimally allowing the foam 60 and ozonated blood 54 to separate substantially adequately, which may help reduce the procedural time and aid the operator in completing the procedure.

As shown in FIG. 7D of the present disclosure, a method for managing foam 700 of the present disclosure additionally comprises at step 2000, the step of ozonating blood 50 by injecting ozone under pressurization. In some such, examples the blood 50 is ozonated under a pressure of at least one bar. In some such, examples the blood 50 is ozonated under a combined pressure of at least one and a half bars.

In some embodiments of the present disclosure, a method for managing foam 700 of ozonating blood is provided where the method uses the foam management apparatus 300 as disclosed herein. In some embodiments, a method is disclosed for ozonating blood, the method using the foam management system as disclosed herein. In additional embodiments of the present disclosure a method is disclosed of treating a patient with sepsis using ozonation, using the foam management system of the present disclosure as disclosed hereinabove.

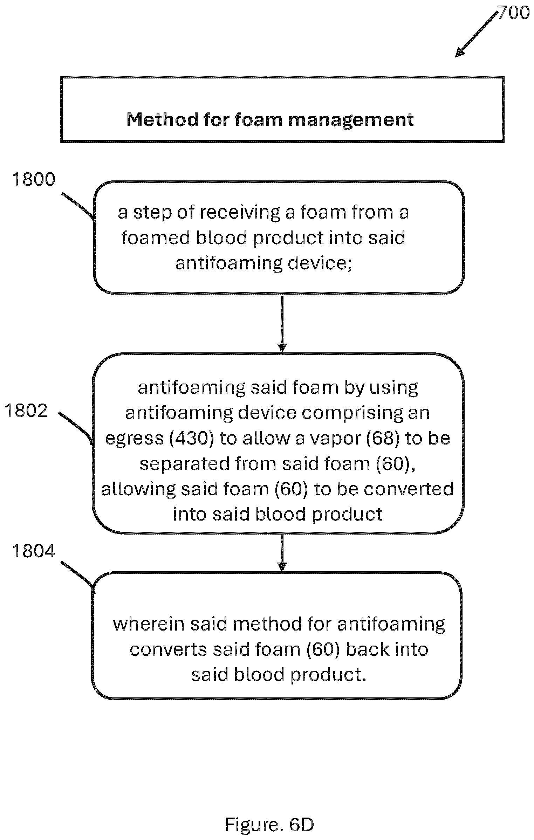

In some embodiments of the present disclosure, as shown in FIG. 6D, a foam management method 700 is disclosed for foam management using an antifoaming device 400, the foam removal method comprises: a step of receiving a foam from a foamed blood product into said antifoaming device at step 1800, antifoaming said foam through an egress 430 of the antifoaming device 400 to allow a gas 68 to be separated from said foam 60 and allowing said foam 60 to be converted into said blood product at step 1802, wherein said method for antifoaming converts said foam back into said blood product at step 1804. In some examples of the method of the present invention, in such embodiments, the foam 60 comprises microbubbles as outlined in the present disclosure as described herein.

### Software

Software and computer readable medium as disclosed and described herein, in accordance with some embodiments of the present disclosure, with reference to drawings enclosed herewith:
In some additional examples of the present disclosure, as shown in FIG. 1, FIG.2, FIG.3, and FIG. 4 and FIG. 5A and FIG. 5B, a software 502 is disclosed for managing foam that provides for both defoaming and antifoaming the foamed ozonated blood 52, that includes code segments for performing the methods of the present disclosure, as disclosed hereinabove. In further examples of the present disclosure, a computer readable medium 501 is disclosed as illustrated in FIGS. 1 to 4, and FIGS. 5A and 5B, where the computer readable medium 501 comprises having stored thereon the software 502 in accordance with examples of the present disclosure, for processing by one or more processing units 500.

Thus, in one broad aspect, a foam management apparatus and/or system is disclosed comprising: antifoaming device for receiving a pressurized foam from a pressurized foamed blood product, said antifoaming device allowing a gas to be separated from said foam, wherein said antifoaming device converts said pressurized foam into said blood product, allowing the blood product to be recovered from said foam, substantially without the foam.

As a feature of this broad aspect, the foam management apparatus and/or system further comprises: a defoaming device for receiving a pressurized foamed blood product, for separating a pressurized foam therefrom for processing, to enable return of the blood product to a person, substantially without the foam. In some examples of the foam management apparatus, the pressurized foam is passively separated into gas and the blood product. In addition to some such examples or in alternative to, the foam comprises microbubbles, wherein said microbubbles are dissolved substantially in said blood product.

As another feature of this broad aspect, the foam management apparatus and/or system further comprises: a chamber defined by the defoaming device, wherein the chamber is for receiving a foamed blood product comprising a blood that is converted into pressurized foamed ozonated blood, for separating a foam therefrom for processing, to enable return of the blood product comprising a primary ozonated blood recovered from said foamed ozonated blood to the body, said foam being pressurized, wherein said chamber separates the pressurized foam and the primary ozonated blood ; and an egress defined by the antifoaming device, wherein the antifoaming device is for receiving said pressurized foam, said egress of the antifoaming device, for allowing a gas to be separated from said foam, allowing said foam to be converted into the blood product comprising a secondary ozonated blood, the secondary ozonated blood being more ozonated than primary ozonated blood; wherein said blood product comprising said primary ozonated blood and said secondary ozonated blood, is returned to the body substantially without the foam, allowing substantially all of the blood processed by said foam management apparatus to be received as ozonated blood, substantially without the foam.

As another feature of this broad aspect, the foam comprises microbubbles of ozone containing gas, wherein said microbubbles are dissolved substantially in said foamed ozonated blood.

As another feature of this broad aspect, the foam management apparatus and/or system further comprises: one or more pumps and/or one or more injectors to pressurize said blood, resulting in the foamed ozonated blood.

As another feature of this broad aspect of the foam management apparatus and/or system, wherein said one or more pumps comprises a peristaltic pump, and wherein said one or more pumps and said one or more injectors have a combined pressure of about 1.5 bars.

As another feature of this broad aspect of the foam management apparatus and/or system of the present disclosure, wherein the antifoaming device egress is defined by an aperture to allow the air or gas to escape, thereby relieving the pressures in the apparatus and/or system, and a valve that enables air or gas to leave the antifoaming device but prevents any foreign matter from entering the apparatus and/or system.

In some such examples, the egress is defined by a needle forming an aperture or shunt. In other examples, the egress is defined by a window. In both cases, a valve such as a check valve is provided that allows the gas to escape, relieving the pressure in the system but prevents foreign matter from entering the system.

As another feature of this broad aspect of the foam management apparatus and/or system of the present disclosure, said one or more pumps and said one or more injectors have a combined pressure of about 1.5 bars.

As another feature of this broad aspect of the foam management apparatus and/or system of the present disclosure, wherein said defoaming device further comprises: one or more sensors for checking a level of the ozonated blood in said chamber; wherein if there is mixing of ozonated blood and foam and/or the level of ozonated blood is low enabling mixing of foam and ozonated blood, a pressure of the foamed ozonated blood can be increased by increasing the pressure in the one or more pumps and/or the injectors, thereby increasing level of ozonated blood in the chamber.

In another broad aspect, a foam management system is disclosed for managing a foamed blood product, the system comprising: the foam management apparatus as disclosed herein; and one or more processing units to operate said one or more of, the one or more pumps, the one or more injectors, the defoaming device, the antifoaming device, and the one or more sensors; thereby separating said foam from said blood product and converting said foam back into said blood product, allowing substantially all of the blood product to be recovered.

As a feature of this broad aspect, the foam management system of the present disclosure, wherein said one or more processing units allow for automated system for control of one or more of: the foam management apparatus; the one or more pumps; the one or more injectors; the defoaming device; the antifoaming device; and the one or more sensors; thereby facilitating managing of foam to allow ease of operation by the operator as well as allowing for automatically adjusting defoaming and antifoaming to optimize return of blood product back to the patient, substantially without the foam.

As another feature of this broad aspect, a foam management method is disclosed using a foam management apparatus of the present disclosure, comprising the steps of: Pressurizing a blood product, wherein said blood product comprises blood, by pumping the blood into pressurized blood; ozonating blood by injecting ozone under pressurization, forming a foamed blood product comprising foamed ozonated blood; receiving foamed ozonated blood into the defoaming device; defoaming the foamed ozonated blood in the chamber of the defoaming device, by separating the foamed ozonated blood into ozonated blood and foam, comprising pressurized foam; antifoaming the pressurized foam by processing said pressurized foam to convert it into secondary ozonated blood, the secondary ozonated blood having more ozonation than primary ozonated blood; and returning primary ozonated blood and the secondary ozonated blood substantially without the foam, allowing substantially all of the blood to be returned to the patient in the form of ozonated blood.

As another feature of this broad aspect of the foam management method of the present disclosure, the method further comprises the steps of: receiving pressurized foam into the antifoaming from the defoaming device; permitting gas to exit from the egress of antifoaming device, to allow the secondary ozonated blood and gas to separate, wherein the secondary ozonated blood remains in the antifoaming device; and returning the secondary ozonated blood, substantially without the foam.

As another feature of this broad aspect of the foam management method of the present disclosure, wherein the method further comprises the steps of: sensing a level of ozonated blood in the chamber or defoaming chamber of the defoaming device; checking if there is mixing of foam and the ozonated blood and/or if the level of ozonated blood is too low enabling mixing of foam and the ozonated blood; controlling the one or more pumps and/or the injectors to increase the pressure therein, to increase a pressure of the foamed ozonated blood entering the defoaming device, thereby substantially preventing mixing of said ozonated blood and the foam; and maintaining a pressure of said pressurized foamed ozonated blood by injecting ozone under pressurization and/or pressurizing the blood at a combined pressure of at least about 1.5 bar.

As still another feature of this broad aspect of the foam management method of the present disclosure, the method further comprises a step of: receiving foamed and/or pressurized ozonated blood directly from a patient, wherein the ozonated blood and the secondary ozonated blood, are both returned to the patient in real time, while the foam management apparatus is connected to the patient.

As still another feature of this broad aspect of the foam management method of the present disclosure, the method further comprises a step of: receiving foamed and/or pressurized ozonated blood from a person, where the person may be the a person other than the patient, wherein the ozonated blood and the secondary ozonated blood, are both returned to a patient via one or more blood bags, while the foam management apparatus is offline or disconnected from the person and/or the patient.

As still another feature of this broad aspect, the foam management method comprises a step of: receiving foamed and/or pressurized ozonated blood from a blood bag; wherein the ozonated blood and the secondary ozonated blood, are both returned to one or more blood bags, while the foam management apparatus is offline. In some such examples, blood is received from a person, which in some cases may or may not comprise the patient, and ozonated and pressurized via one more ozone injectors, for injecting ozone into the blood and one or more pumps for pressurizing the blood, thereby forming foamed and/or pressurized ozonated blood, that is stored in said blood bag, prior to receiving foamed and/or pressurized ozonated blood from said blood bag.

In another broad aspect of the present disclosure, a software is disclosed for carrying out the method and/ procedure of ozonating blood as defined by the apparatus and/or system of the present disclosure, wherein the software includes code segments for performing the method of foam management of the present disclosure, wherein said method is preferably automated. Alternatively and/or additionally in some examples, a computer readable medium is disclosed comprising having stored thereon the software, for processing by one or more processing units preferably in a system of the present disclosure. In some such examples, the method is automated.

In accordance with embodiments of the method of the present disclosure, any of the methods described with reference to foamed ozonated blood or ozonated blood as described herein above with respect to defoaming and antifoaming, can be performed with any other foamed blood product, such as foamed oxygenated blood or oxygenated blood, as an example, for defoaming and antifoaming oxygenated blood. The methods disclosed herein above can also be used with respect to defoaming and antifoaming any foamed blood product and/or components thereof, as may be known in the art.

Embodiments of the present disclosure are described herein with reference to flowchart and/or block diagrams. It will be understood that some or all of the illustrated blocks may be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function/act specified in the flowchart and/or block diagram block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the flowchart and/or block diagram block or blocks.

The present disclosure has been described above with reference to specific embodiments. However, other embodiments than the above described are equally possible within the scope of the disclosure. Different method steps than those described above, performing the method by hardware or software, may be provided within the scope of the disclosure. The different features and steps of the disclosure may be combined in other combinations than those described. The scope of the disclosure is only limited by the appended patent claims.

### REFERENCE LIST

(1000) foam management system
   (300) foam management apparatus
      (100) one or more pumps
      (160) peristaltic or tube pump
      (150) one or more injectors
      (50) blood received from the patient
      (51) pressurized blood from the pump
      (52) foamed ozonated blood
      (54) ozonated blood for return to the patient
      (56) secondary ozonated blood (which is more ozonated than ozonated blood)
      (60) foam
      (64) Ozone or Ozone gas
      (200) defoaming device
         (202) chamber
         (220) sensors
         (400) antifoaming device
            (430) air egress or vent
            (431) needle
            (432) shunt or aperture
            (434) window
            (436) valve
            (68) gas or air
(500) Processing unit
   (501) Computer readable medium
   (502) software
(600) one or more blood bags
(605) a first blood bag
(610) intermediate blood bag
(620) a second blood bag
(700) method for foam management
Steps (1800) to (1804) method steps
Steps (2000) to (2018) method steps
(800) IV drip chamber
(820) IV clamp or pinch

## Claims

1. A foam management apparatus (300) comprising:
antifoaming device (400) for receiving a pressurized foam (60) from a pressurized foamed blood product, said antifoaming device allowing a gas (68) to be separated from said foam (60), wherein said antifoaming device converts said pressurized foam (60) into said blood product, allowing the blood product to be recovered from said foam (60), substantially without the foam.

2. The foam management apparatus (300) of claim 1, further comprising:
a defoaming device (200) for receiving a pressurized foamed blood product, for separating a pressurized foam (60) therefrom for processing, to enable return of the blood product, substantially without the foam.

3. The foam management apparatus (300) of claim 2, wherein the pressurized foam (60) is passively separated into gas (68) and the blood product, and/or wherein said foam comprises microbubbles, wherein said microbubbles are dissolved substantially in said blood product.

4. The foam management apparatus (300) of claim 3, further comprising:
a chamber (202) defined by the defoaming device (200);
wherein the chamber 202 is for receiving a foamed blood product comprising a blood (50) that is converted into pressurized foamed ozonated blood (52), for separating a foam (60) therefrom for processing, to enable return of the blood product comprising a primary ozonated blood (54) recovered from said foamed ozonated blood (52) to the body, said foam (60) being pressurized, wherein said chamber (202) separates the pressurized foam (60) and the primary ozonated blood (54);
an egress (430) defined by the antifoaming device (400); and
wherein the antifoaming device (400) is for receiving said pressurized foam (60), said egress (430) of the antifoaming device (400), for allowing a gas (68) to be separated from said foam (60), allowing said foam (60) to be converted into the blood product comprising a secondary ozonated blood (56), the secondary ozonated blood (56) being more ozonated than primary ozonated blood (54);
wherein said blood product comprising said primary ozonated blood (54) and said secondary ozonated blood (56), is returned to the body substantially without the foam, allowing substantially all of the blood (50) processed by said foam management apparatus (300) to be received as ozonated blood (54,56), substantially without the foam.

5. The foam management apparatus of claim 4, wherein the foam (60) comprises microbubbles of ozone containing gas, wherein said microbubbles are dissolved substantially in said foamed ozonated blood (52).

6. The foam management apparatus (300) of claim 5, further comprising:
one or more pumps (100) and/or one or more injectors (150) to pressurize said blood (50), resulting in the foamed ozonated blood (52).

7. The foam management apparatus (300) of claim 6, wherein said one or more pumps (100) comprises a peristaltic pump (160) and wherein said one or more pumps (100) and said one or more injectors (150) have a combined pressure of about 1.5 bars.

8. The foam management apparatus (300) of claim 7, wherein the antifoaming device egress (430) is defined by an aperture (432) to allow the air or gas (68) to escape, thereby relieving the pressures in the apparatus, and a valve (436) that enables air or gas (68) to leave the antifoaming device (400) but prevents any foreign matter from entering the apparatus.

9. The foam management apparatus (300) of claim 8, wherein said defoaming device (200) further comprises:
a sensor (220) for checking a level of the ozonated blood (54) in said chamber (202);
wherein if there is mixing of ozonated blood (54) and foam (60) and/or the level of ozonated blood (54) is low enabling mixing of foam (60) and ozonated blood (54), a pressure of the foamed ozonated blood (52) can be increased by increasing the pressure in the one or more pumps (100) and/or the injectors (150), thereby increasing level of ozonated blood in the chamber 202.

10. A foam management system (1000) for managing a foamed blood product, the system comprising one or more pumps (100), one or more injectors (150), a defoaming device (400) and a sensor (220):
the foam management apparatus (300) of any one of claims 1 to 9; and
one or more processing units (500) to operate said one or more of, the one or more pumps (100), the one or more injectors (150), the defoaming device (200),
the antifoaming device (400), and the sensor (220);
thereby separating said foam (60) from said blood product and converting said foam back into said blood product, allowing substantially all of the blood product to be recovered.

11. The foam management system (1000) of claim 10,
wherein said one or more processing units (500) allow for automated system for control of one or more of:
the foam management apparatus (300);
the one or more pumps (100);
the one or more injectors (150);
the defoaming device (200),
the antifoaming device (400);
and the sensor (220);
thereby facilitating managing of foam to allow ease of operation by the operator as well as allowing for automatically adjusting defoaming and antifoaming to optimize return of blood product, substantially without the foam.

12. A foam management method using a foam management apparatus (300) of any one of claims 1 to 9, comprising the steps of:
- Pressurizing a blood product, wherein said blood product comprises blood (50), by pumping the blood (50) into pressurized blood (51) at a first step (2000);
- ozonating blood (50) by injecting ozone under pressurization, forming a foamed blood product comprising foamed ozonated blood (52), at step (2000);
- receiving foamed ozonated blood (52) into the defoaming device (200), at step (2002);
- defoaming the foamed ozonated blood (52) in the chamber (202) of the defoaming device (200), at step (2004), by separating the foamed ozonated blood (52) into ozonated blood (54) and foam (60), comprising pressurized foam (60);
- antifoaming the pressurized foam (60) by processing said pressurized foam (60) to convert it into secondary ozonated blood (56), the secondary ozonated blood (56) having more ozonation than primary ozonated blood (54), at step (2005), and returning primary ozonated blood (54) and the secondary ozonated blood (56), at step (2006), substantially without the foam (60), allowing substantially all of the blood (50) to be returned in the form of ozonated blood (54, 56);
further comprising a step of either:
- receiving foamed ozonated blood (52) from a blood bag (600) when receiving foamed ozonated blood (52) into the defoaming device (200), wherein the ozonated blood (54) and the secondary ozonated blood (56), are both returned to one or more blood bags (600), while the foam management apparatus (300) is offline; or
- receiving foamed ozonated blood (52) directly from a patient when receiving foamed ozonated blood (52) into the defoaming device (200), wherein the ozonated blood (54) and the secondary ozonated blood (56), are both returned to the patient in real time, while the foam management apparatus (300) is connected to the patient; or
- receiving foamed ozonated blood (52) from a person, when receiving foamed ozonated blood (52) into the defoaming device (200), wherein the ozonated blood (54) and the secondary ozonated blood (56), are both returned to a patient via one or more blood bags (600), while the foam management apparatus (300) is offline or disconnected.

13. The foam management method of claim 12, wherein at step (2005), the method further comprises the steps of:
- receiving pressurized foam (60) into the antifoaming device (400) from the defoaming device (200) at step (2008);
- permitting gas to exit from the egress (430) of antifoaming device (400), to allow the secondary ozonated blood (56) and gas (68) to separate, wherein the secondary ozonated blood (56) remains in the antifoaming device (400), at step (2010); and
returning the secondary ozonated blood (56), substantially without the foam.

14. The foam management method of claim 13, wherein the method further comprises the steps of:
- sensing a level of ozonated blood (54) in the chamber (202), at step (2016);
- checking if there is mixing of foam (60) and the ozonated blood (54) and/or if the level of ozonated blood (54) is too low enabling mixing of foam (60) and the ozonated blood (54);
- controlling the one or more pumps (100) and/or the injectors (150) to increase the pressure therein, to increase a pressure of the foamed ozonated blood (52) entering the defoaming device (200), thereby substantially preventing mixing of said ozonated blood (54) and the foam (60); and
- maintaining a pressure of said pressurized foamed ozonated blood (52) by injecting ozone under pressurization and/or pressurizing the blood at a combined pressure of at least about 1.5 bar, at step (2000).

15. A software for ozonating blood, that includes code segments for performing the method of any of claims 12 to 14 for foam management wherein said method is preferably automated; or
a computer readable medium (501) having stored thereon said software for processing by one or more processing units (500) preferably in a system of any of claims 10 or 11.
